# EUROPEAN PATENT APPLICATION

(11) **EP 4 378 953 A1**
(43) Date of publication of application: **05.06.2024**
(21) Application number: 22383180.1
(22) Date of filing: 02.12.2022
(51) Int. Cl.: C07K 16/28, A61P 35/00, C07K 14/725, C12N 5/0783, A61K 39/00

(54) **CD229 TARGETING MOIETY FOR THE TRATMENT OF CD229 POSITIVE CANCER**

(71) Applicant: Universitat de Barcelona, 08028 Barcelona (ES); Institut d'Investigacions Biomèdiques August Pi i Sunyer, 08036 Barcelona (ES); Hospital Clínic de Barcelona, 08036 Barcelona (ES)
(72) Inventor: ENGEL ROCAMORA, Pablo, 08036 Barcelona (ES); RODRÍGUEZ LOBATO, Luis Gerardo, 08036 Barcelona (ES); FERNÁNDEZ DE LARREA RODRÍGUEZ, Carlos José, 08036 Barcelona (ES); CARDÚS GRANELL, Oriol, 08036 Barcelona (ES)
(74) Representative: Hoffmann Eitle

(57) **Abstract**

The present invention relates to a CD229 targeting moiety, wherein the CD229 targeting moiety is an antibody, F(ab')2, Fab, scFab or scFv. The present invention further provides CARs, nucleic acid, cells, pharmaceutical compositions and kits comprising the CD229 targeting moiety. Lastly, methods of treatment of a CD229-positive cancer, preferably Multiple Myeloma, are also provided.

## Description

### TECHNICAL FIELD

The present invention provides therapeutics for the treatment of CD229-positive cancers. In particular, the present invention provides a CD229 targeting moiety.

### BACKGROUND ART

Multiple myeloma (MM) is a clonal plasma cell (PC) malignancy that develops in the patients' bone marrow (BM) and ultimately causes bone lesions with fractures, kidney failure, BM failure, and immunoparesis with fatal infections. The standard of care for patients with MM includes treatments such as proteasome inhibitors, immunomodulatory drugs, autologous stem cell transplantation, and monoclonal antibodies, which have substantially prolonged patient survival. However, to date, MM still remains an incurable disease. Chimeric antigen receptor (CAR) T cells have emerged as an effective approach for a number of other hematologic malignancies and recent results from BCMA-targeting CAR T-cell clinical trials in MM indeed showed impressive overall response rates.

B-cell maturation antigen (BCMA) is the predominantly used target in CAR therapies against MM. BCMA target choice is based on its high expression on the surface of malignant plasma cells and restricted expression in normal tissues/cells with the known exception of mature B-cells. However, many other targets have been explored and are emerging from preclinical studies in the context of adoptive cell therapies (ACTs) for MM. Most studies employed anti-BCMA targeting either alone, or in combination with a second target. The second target most studied was SLAMF7 (CD319) explored by clinical trials. Other antigens investigated and described here include: CD44v6, CD38, CD138, MUC1, CD56, CD19, Igk light chain, Lewis Y, CD229 and GPRC5D.

CD229/LY9 is a cell surface receptor present on B and T lymphocytes that is universally and strongly expressed on MM plasma cells. A CAR-based therapy has been developed against CD229, showing remarkable activity. However, there is still not a defined CART based therapy that works in all patients with low toxicity and side effects. Thus, efforts to develop new CARs against new targets are still needed.

### SUMMARY OF INVENTION

In a first aspect, the present invention relates to a CD229 targeting moiety, wherein the CD229 targeting moiety is an antibody, F(ab')2, Fab, scFab or scFv, comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 consists of SEQ ID NO: 4 [SSVSY],
- LCDR2 consists of SEQ ID NO: 5 [DTS],
- LCDR3 consists of SEQ ID NO: 6 [CQQWSSYPPTF],
- HCDR1 consists of SEQ ID NO: 7 [GYTFTHYY],
- HCDR2 consists of SEQ ID NO: 8 [IFPESGST], and
- HCDR3 consists of SEQ ID NO: 9 [CARGTGFFDYW].

Preferably, CD229 targeting-moiety comprises:
- a VL domain comprising a sequence with at least 85%, 90%, 92%, 94%, preferably 95%, 98%, or 100% sequence identity to SEQ ID NO: 1, and wherein said VL domain comprises CDR regions consisting of LCDR1 as set forth in SEQ ID NO: 4, LCDR2 as set forth in SEQ ID NO: 5, LCDR3 as set forth in SEQ ID NO: 6, and wherein
- a VH domain comprising a sequence with at least 85%, 90%, 92%, 94%, preferably 95%, 98%, or 100% sequence identity to SEQ ID NO: 2, and wherein said VH domain comprises CDR regions consisting of HCDR1 as set forth in SEQ ID NO: 7, HCDR2 as set forth in SEQ ID NO: 8, and HCDR3 as set forth in SEQ ID NO: 9.

Preferably, the VL domain comprises a sequence with at least 85%, 90%, preferably 95% sequence identity to SEQ ID NO: 1, and wherein said VL domain comprises CDR regions consisting of LCDR1 as set forth in SEQ ID NO: 4, LCDR2 as set forth in SEQ ID NO: 5, LCDR3 as set forth in SEQ ID NO: 6, and the VH domain comprises a sequence with at least 85%, 90%, preferably 95% sequence identity to SEQ ID NO: 2, wherein said VH domain comprises CDR regions consisting of HCDR1 as set forth in SEQ ID NO: 7, HCDR2 as set forth in SEQ ID NO: 8, and HCDR3 as set forth in SEQ ID NO: 9.

Preferably, the CD229 targeting-moiety is an antibody, F(ab')2, Fab, scFab or scFv, comprising a VL domain and VH domain, wherein the VL domain consists of SEQ ID NO: 1 and the VH domain consists of SEQ ID NO: 2. Preferably, the CD229 targeting-moiety is an scFV, more preferably a scFV consisting of SEQ ID NO: 17.

In a preferred embodiment, the first aspect provides a CAR comprising:
- an extracellular domain comprising a CD229 targeting-moiety, wherein the CD229 targeting-moiety is as defined in the first aspect or any of its embodiments;
- a transmembrane domain; and
- an intracellular signaling domain.

Preferably, the transmembrane domain comprises the transmembrane domain of CD8. Preferably, the intracellular signaling domain comprises the intracellular domain of CD3ζ. Preferably, the costimulatory signaling domain comprises the intracellular domain of CD137.

Preferably, the CAR consists of SEQ ID NO: 18.

A second aspect provides a nucleic acid encoding the CAR according to the first aspect.

A third aspect provides a cell, preferably a T-cell, comprising the nucleic acid according to the second aspect.

A fourth aspect provides a CD229 targeting-moiety according to any of the previous aspects, for use in a method of treating a CD229-positive cancer, wherein the method comprises administering the CD229 targeting moiety, the CAR, the nucleic acid, or the cell to a patient in need thereof, and wherein the CD229-positive cancer is Multiple Myeloma. Preferably, said use is in combination with an anti BCMA therapy, more preferably wherein the anti BCMA therapy comprises administering a CAR against BCMA.

### BRIEF DESCRIPTION OF DRAWINGS

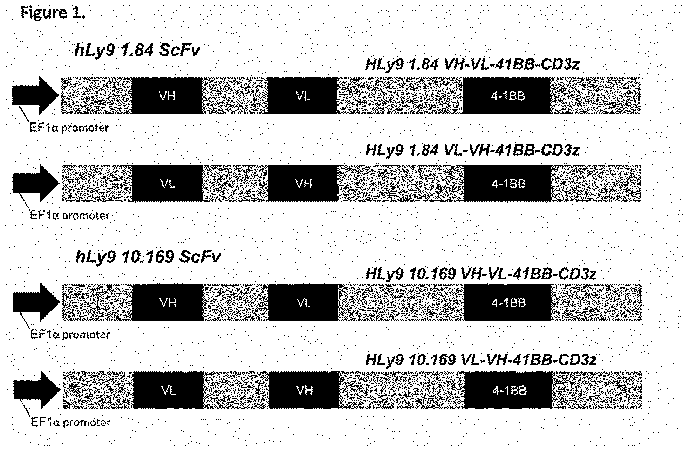

**Fig. 1****: Diagram of the four anti-CD229 CAR constructs.** hLy9 1.84 antibody was used to generate VH-VL-41BB-CD3z (GL001) and VL-VH-41BB-CD3z (GL002) CARs. hLy 10.169 was used to generate VH-VL-41BB-CD3z (GL005) and VL-VH-41BB-CD3z (GL006) CARs.

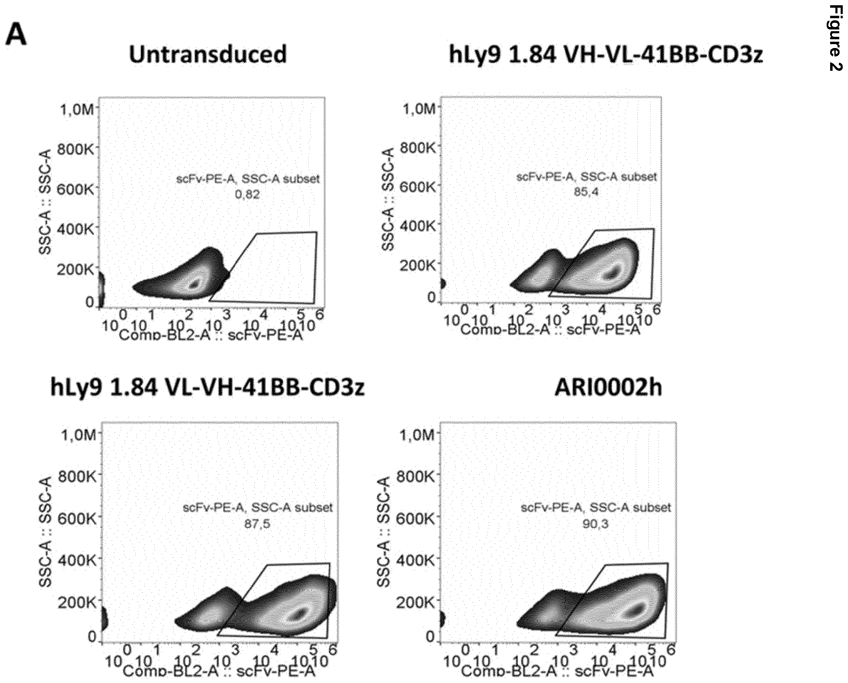

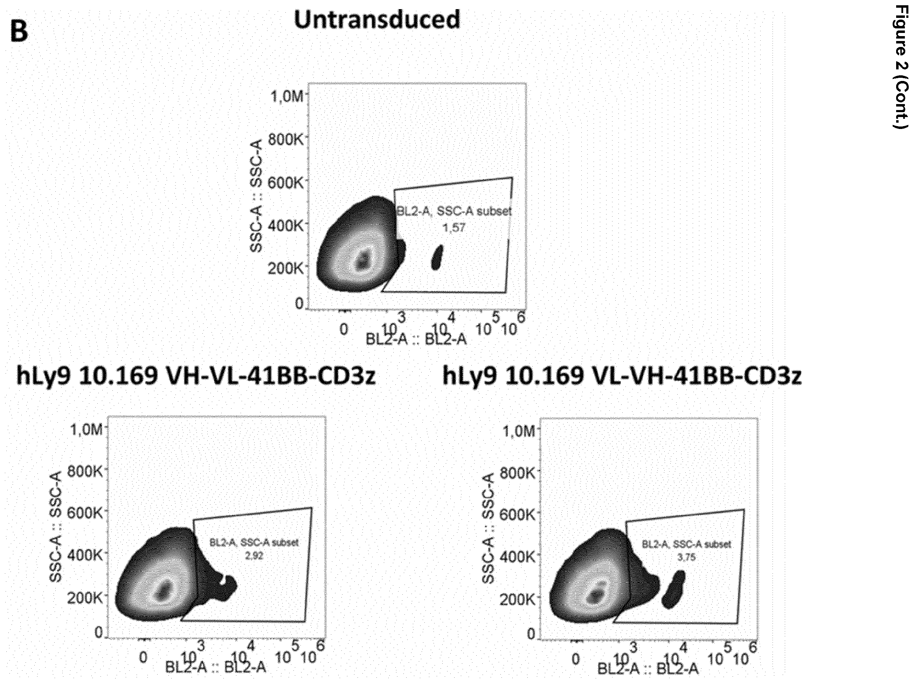

**Fig. 2****: Detection of CAR protein on the cell surface.** A. Detection of GL001 (hLy9 1.84 VH-VL-41BB-CD3z), GL002 (hLy9 1.84 VL-VH-41BB-CD3z), and ARI002h (anti-BCMA) on the surface of T cells (both CD4 and CD8). B. Detection of GL005 (hLy9 10.169 VH-VL-41BB-CD3z) and GL006 (hLy9 10.169 VL-VH-41BB-CD3z) on the surface of HEK293-T cells. Detection of the chimeric protein was performed by flow cytometry.

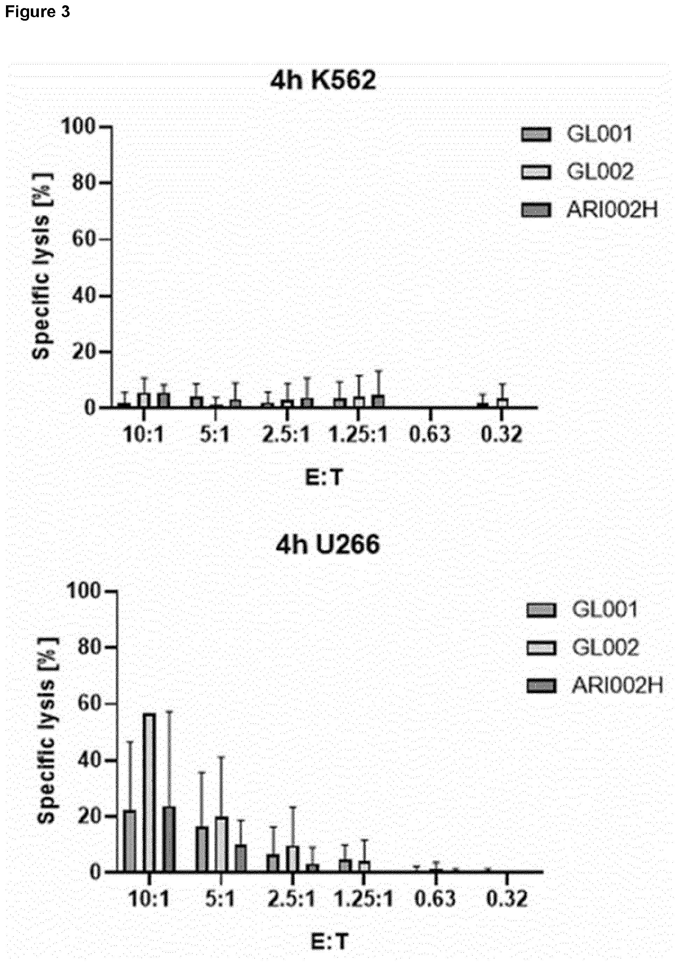

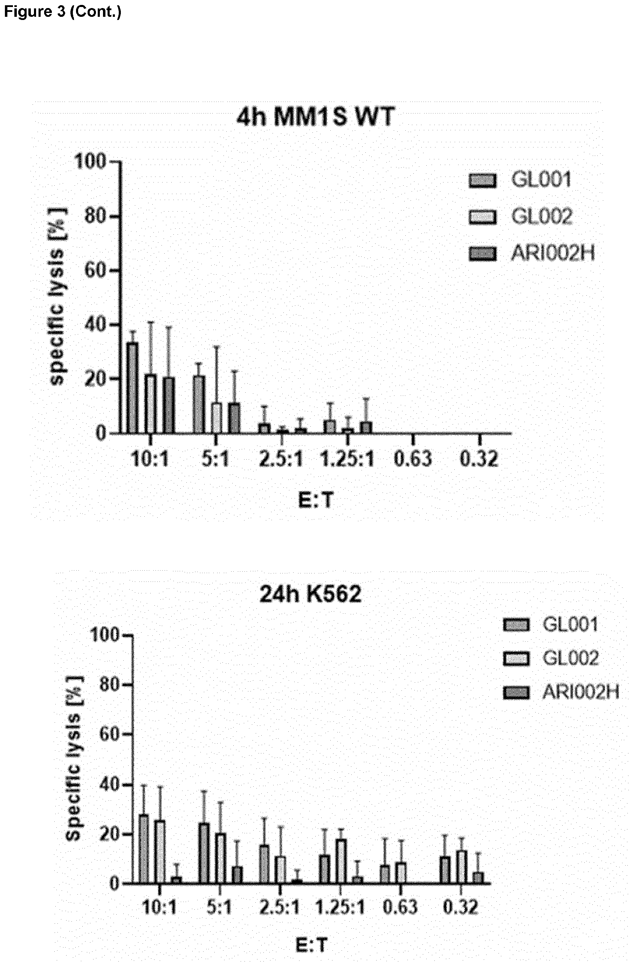

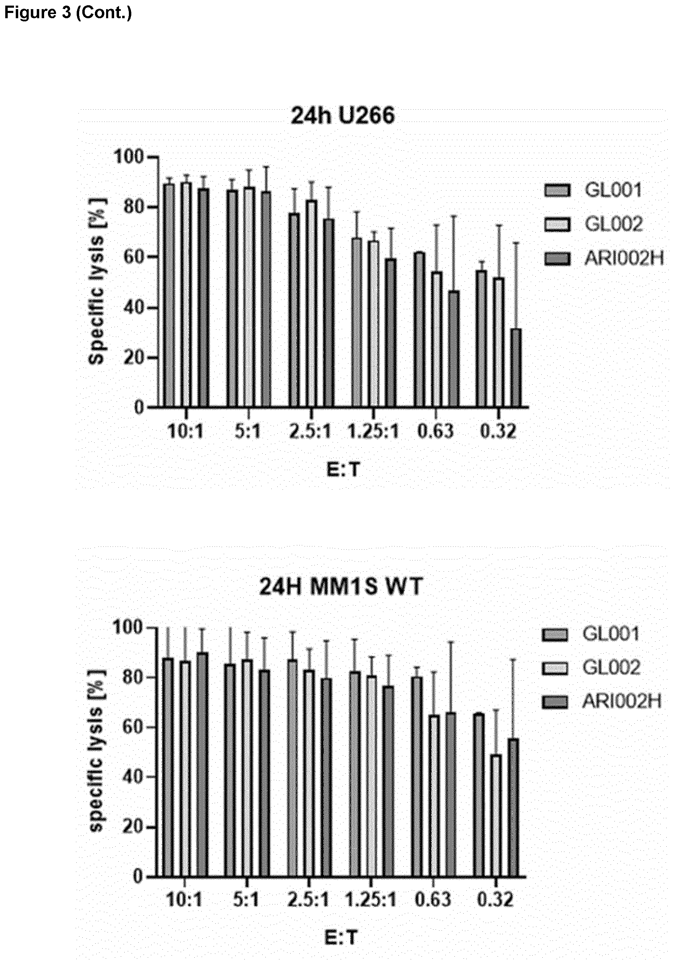

**Fig. 3****: CAR T-cell cytotoxic capacity against different target cells.** Killing capacity of GL001, GL002, and ARI002h CAR-T cells co-incubated at different ratios during 4h and 24h with K562 WT (BCMA- and CD229-), MM.1S WT (BCMA+ and CD229+) and U266 WT (BCMA+ CD229+) tumor cells. The mean of 3 experiments with 3 different donors and ± SD is shown.

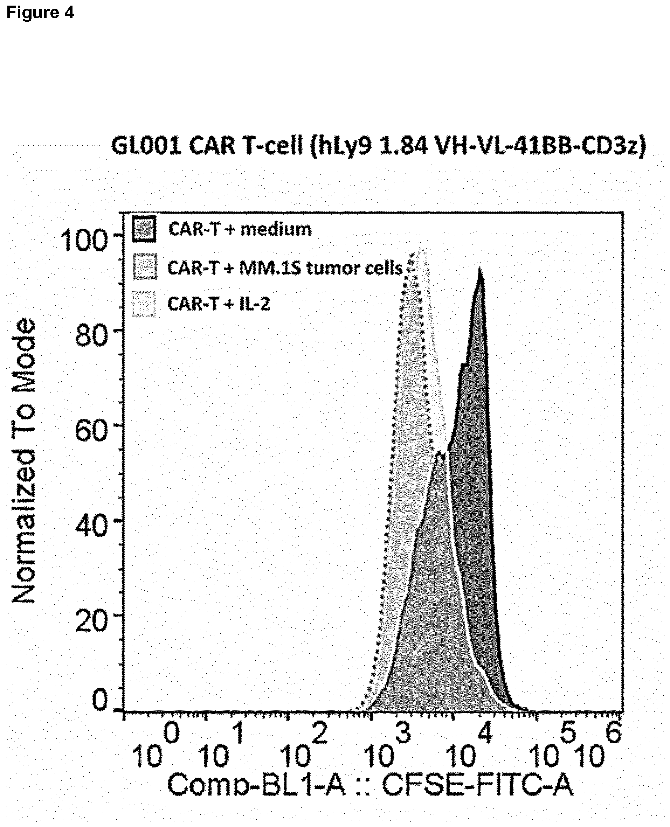

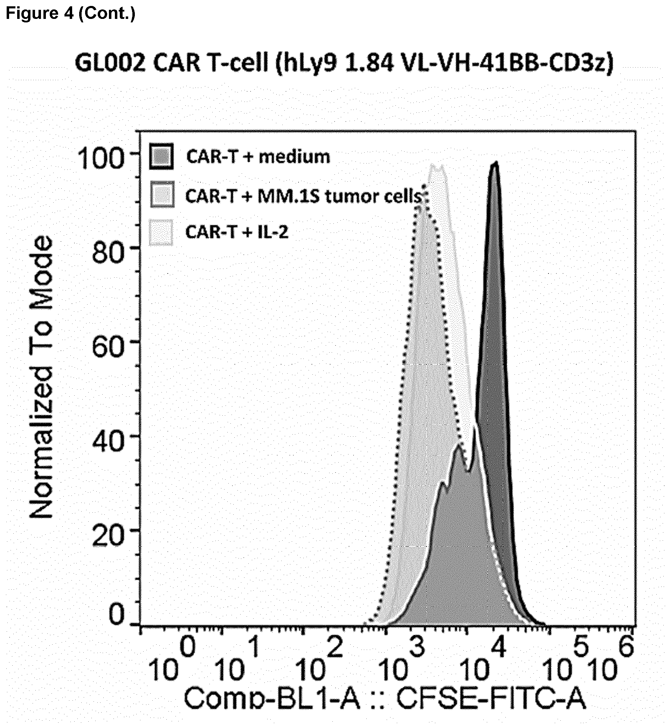

**Fig. 4****: CAR T-cell proliferation.** GL001 (left) and GL002 (right) T-cell proliferation in vitro, measured by CFSE assay at the 72-hr time point. Panels show representative flow cytometry images.

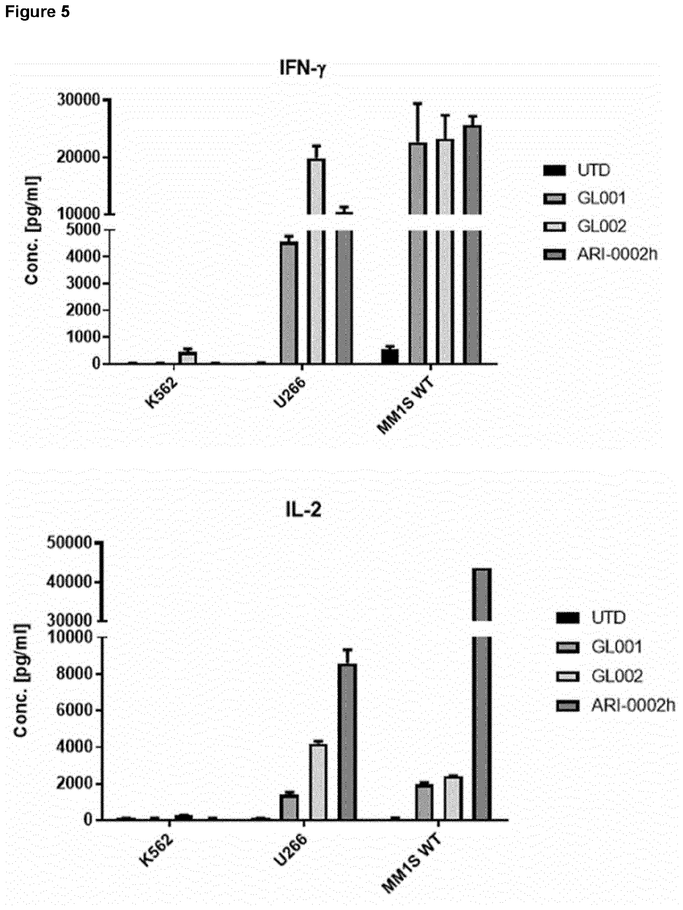

**Fig. 5****: CAR T-cell cytokine secretion.** Cytokine production (IFNg and IL-2) of GL001, GL002 and ARI002h cells in co-culture with K562, MM.1S and U266 cells at the 24-hr time point, measured by ELISA.

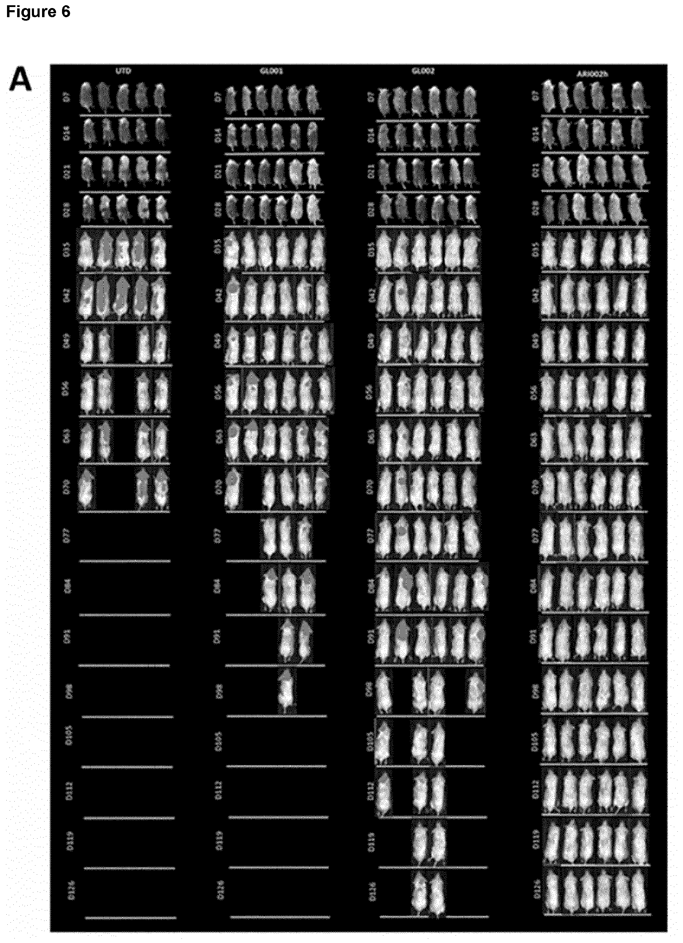

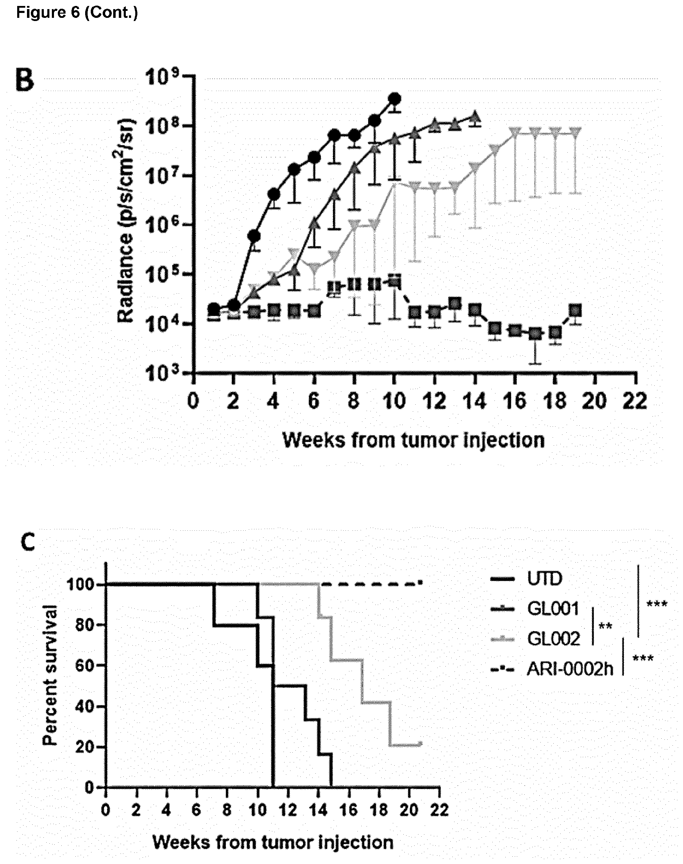

**Fig. 6****: Bioluminiscence imaging showing differences in tumor progression on mice treated with different CAR T-cells (UTD, GL001, GL002, and ARI002h)** Diagram of experimental design and quantification of disease progression by weekly bioluminescence imaging and overall survival of the different groups of mice. In vivo experimental design-mice were injected with 5 × 10⁶ GFP-ffLuc-expressing U266 cells. Day 14, mice were randomized to treatment with 3.0 ×10⁶ untransduced (UTD), GL001, GL002 or ARI0002h. A. Tumor progression was measured by taking weekly bioluminescence images. B. Quantification of tumor burden by bioluminescence imaging over time of tumor cell (U266 WT) infusion. Lines show the median and the 95% confidence interval from all mice in that group. C. Overall survival of mice from each group. * p<0.01; ** p<0.001.

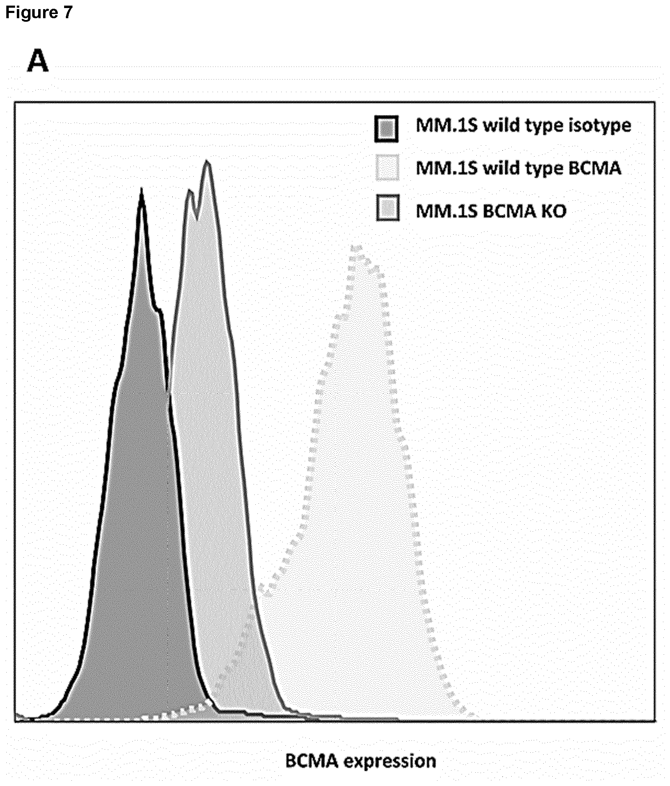

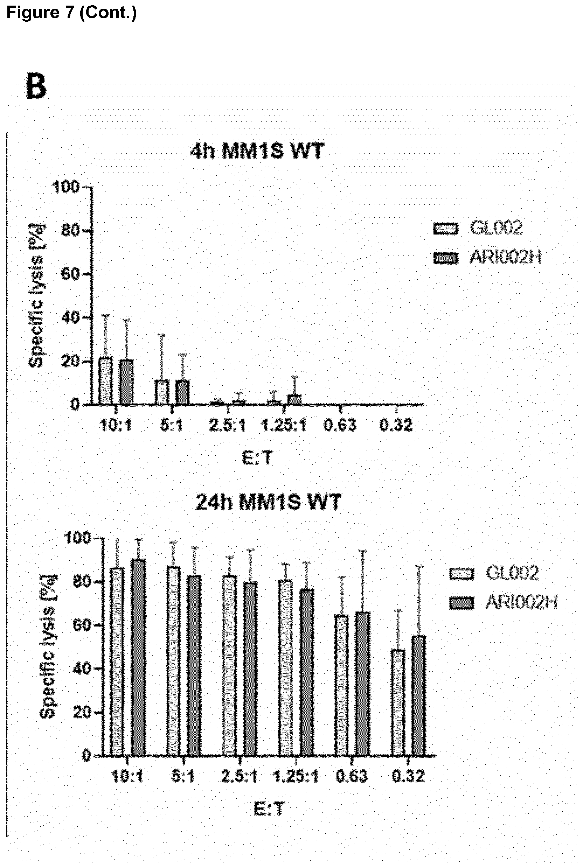

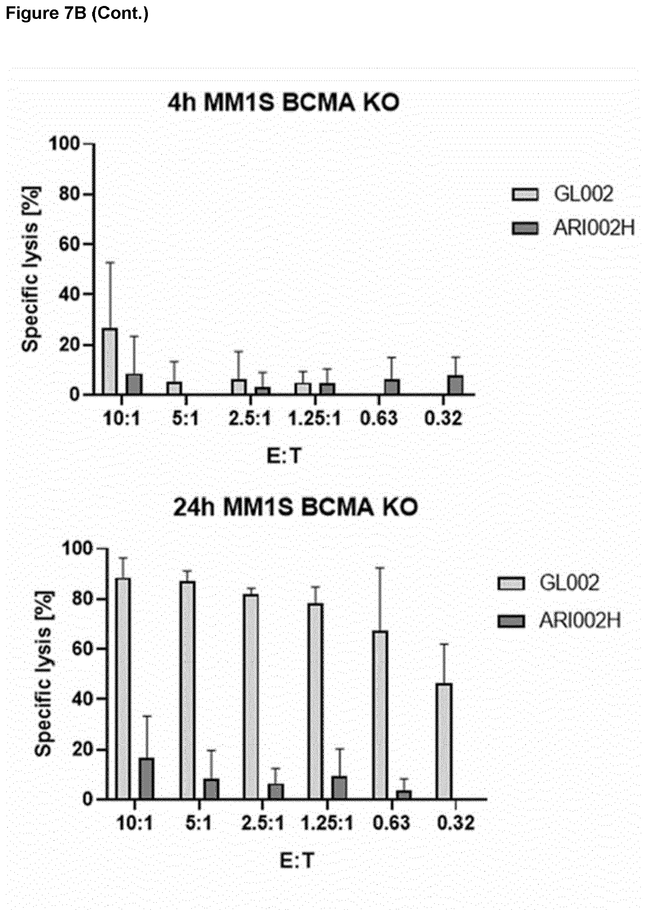

**Fig. 7****: CAR-T cell cytotoxic capacity against target cells with different BCMA antigen expression.** A: Flow cytometry of MM.1S BCMA KO cells, showing BCMA antigen expression. B: Killing capacity of GL002 and ARI002h CAR-T cells co-incubated at different ratios during 4h and 24h with MM.1S WT (BCMA+ and CD229+) and MM.1S BCMA KO (BCMA- CD229+) tumor cells. The mean of 3 experiments with 3 different donors and ± SD is shown.

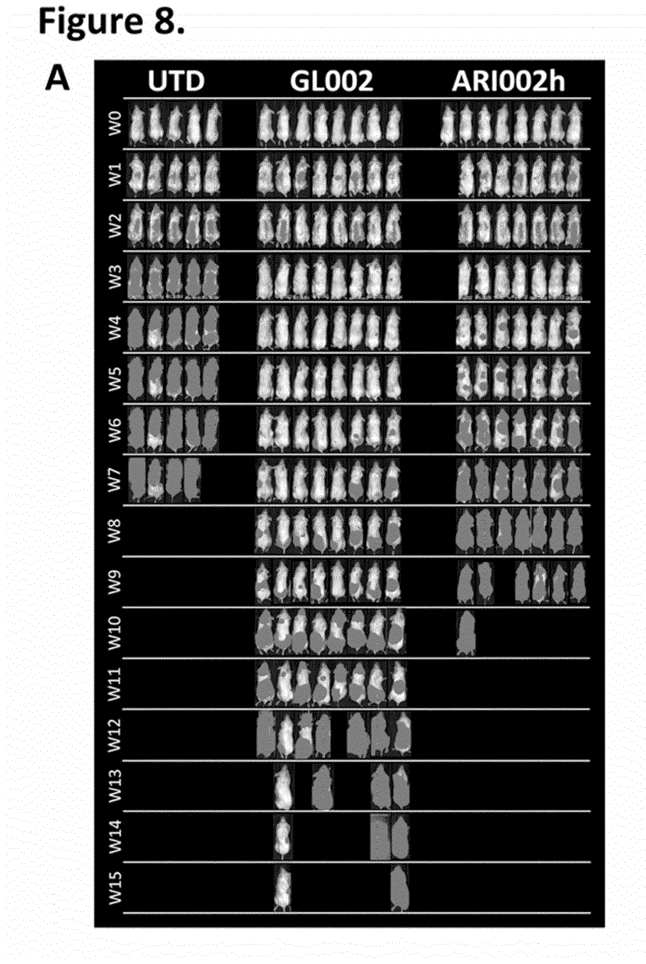

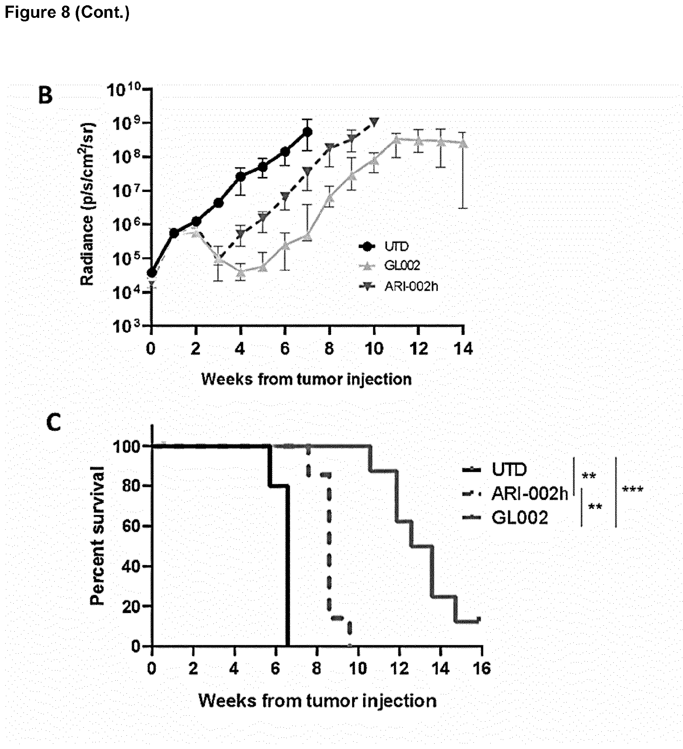

Fig. 8: Bioluminiscence imaging showcasing differences in tumor progression of a mixed population of specific antigen expressing and non-expressing tumor cells on mice treated with different CAR-Ts (UTD, GL002 and ARI002h). In vivo experimental design-mice were injected with 2 × 10⁶ GFP-ffLuc-expressing MM.1S cells, including a population (15%) of BCMA-KO cells. Day 14, mice were randomized to treatment with 1.0 ×10⁶ untransduced (UTD), GL002, or ARI0002h. A. Tumor progression was measured by taking weekly bioluminescence images. B. Quantification of tumor burden by bioluminescence imaging over time of tumor cell (MM.1Swt and MM.1S BCMAKO) infusion. Lines show the median and the 95% confidence interval from all mice in that group. C. Overall survival of mice from each group. ** p<0.001; *** p<0.0001.

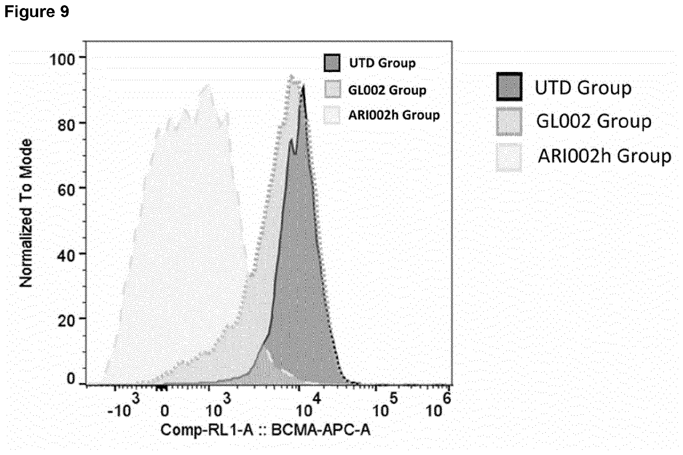

Fig. 9: BCMA antigen expression on bone marrow from mice. BCMA antigen expression on bone marrow MM.1S myeloma tumor cells harvested from mice treated with UTD, GL002, or ARI002h CAR T-cells. Antigen expression detected by flow cytometry.

### GENERAL DEFINITIONS

"Administering" or "administration of" a medicament to a patient (and grammatical equivalents of this phrase) refers to direct administration, which may be administration to a patient by a medical professional, and/or indirect administration, which may be the act of prescribing a drug. E.g., a physician who instructs a patient to self-administer a medicament or provides a patient with a prescription for a drug is administering the drug to the patient.

The term "affibody" refers to a protein that is derived from the Z domain of protein A and that been engineered to bind to a specific target (see Frejd & Kim, 2017. Exp Mol Med. 49(3): e306).

The term "antibody" refers to a molecule comprising at least one immunoglobulin domain that binds to, or is immunologically reactive with, a particular target. The term includes whole antibodies and any antigen binding portion or single chains thereof and combinations thereof; for instance, the term "antibody" in particular includes bivalent antibodies and bivalent bispecific antibodies.

A typical type of antibody comprises at least two heavy chains ("HC") and two light chains ("LC") interconnected by disulfide bonds.

Each "heavy chain" comprises a "heavy chain variable domain" (abbreviated herein as "VH") and a "heavy chain constant domain" (abbreviated herein as "CH"). The heavy chain constant domain typically comprises three constants domains, CH1, CH2, and CH3.

Each "light chain" comprises a "light chain variable domain" (abbreviated herein as "VL") and a "light chain constant domain" ("CL"). The light chain constant domain (CL) can be of the kappa type or of the lambda type. The VH and VL domains can be further subdivided into regions of hypervariability, termed Complementarity Determining Regions ("CDR"), interspersed with regions that are more conserved, termed "framework regions" ("FW"). In molecular biology, a framework region is a subdivision of the variable region (Fab) of the antibody. The variable region is composed of seven regions, four of which are framework regions and three of which are hypervariable regions. The framework region makes up about 85% of the variable region, and are responsible for acting as a scaffold for the CDR regions. These CDRs are in direct contact with the antigen and are involved in binding antigen, while the framework regions support the binding of the CDR to the antigen and may aid in maintaining the overall structure of the four variable domains on the antibody.

Each VH and VL is composed of three CDRs and four FWs, arranged from amino-terminus to carboxy-terminus in the following order: FW1, CDR1, FW2, CDR2, FW3, CDR3, FW4. The present disclosure inter alia presents VH and VL sequences as well as the subsequences corresponding to CDR1, CDR2, and CDR3.

The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme).

Accordingly, a person skilled in the art would understand that the sequences of FW1, FW2, FW3 and FW4 are equally disclosed. For a particular VH, FW1 is the subsequence between the N-terminus of the VH and the N-terminus of H-CDR1, FW2 is the subsequence between the C-terminus of H-CDR1 and the N-terminus of H-CDR2, FW3 is the subsequence between the C-terminus of H-CDR2 and the N-terminus of H-CDR3, and FW4 is the subsequence between the C-terminus of H-CDR3 and the C-terminus of the VH. Similarly, for a particular VL, FW1 is the subsequence between the N-terminus of the VL and the N-terminus of L-CDR1, FW2 is the subsequence between the C-terminus of L-CDR1 and the N-terminus of L-CDR2. FW3 is the subsequence between the C-terminus of L-CDR2 and the N-terminus of L-CDR3, and FW4 is the subsequence between the C-terminus of L-CDR3 and the C-terminus of the VL.

The variable domains of the heavy and light chains contain a region that interacts with a binding target, and this region interacting with a binding target is also referred to as an "antigen-binding site" or "antigen binding site" herein. The constant domains of the antibodies can mediate the binding of the antibody to host tissues or factors, including various cells of the immune system (e.g., effector cells) and the first component (C1q) of the classical complement system. Exemplary antibodies of the present disclosure include typical antibodies, but also bivalent fragments and variations thereof such as a F(ab')2.

As used herein, the term "antibody" encompasses intact polyclonal antibodies, intact monoclonal antibodies, bivalent antibody fragments (such as F(ab')2), multispecific antibodies such as bispecific antibodies, chimeric antibodies, humanized antibodies, human antibodies, and any other modified immunoglobulin molecule comprising an antigen binding site.

The term "humanized antibody" refers to forms of antibodies that contain sequences from non-human (eg, murine) antibodies as well as human antibodies. A humanized antibody can include conservative amino acid substitutions or non-natural residues from the same or different species that do not significantly alter its binding and/or biologic activity. Such antibodies are chimeric antibodies that contain minimal sequence derived from non-human immunoglobulins. For the most part, humanized antibodies are human immunoglobulins (recipient antibody) in which residues from a complementary-determining region (CDR) of the recipient are replaced by residues from a CDR of a non-human species (donor antibody) such as mouse, rat, camel, bovine, goat, or rabbit having the desired properties. Furthermore, humanized antibodies can comprise residues that are found neither in the recipient antibody nor in the imported CDR or framework sequences. These modifications are made to further refine and maximize antibody performance. Thus, in general, a humanized antibody can comprise all or substantially all of at least one, and in one aspect two, variable domains, in which all or substantially all of the hypervariable loops correspond to those of a non-human immunoglobulin and all or substantially all of the FR regions are those of a human immunoglobulin sequence. The humanized antibody optionally also can comprise at least a portion of an immunoglobulin constant region (Fc), or that of a human immunoglobulin. The process to humanize an antibody comprises a CDR-grafting technique. This technique implies the use of a "donor" antibody containing the antigen-specific CDRs and the "acceptor" antibody that will serve as the framework for the hybrid molecule. Usually, the "donor" antibody is the non-human derived antibody, such as the murine antibody, and the "acceptor" antibody is the human antibody. As a result of the humanization process, the resulting humanized antibody or hybrid antibody maintains the CDR sequences, in both the VL and VH domains, of the "donor" antibody, whereas the framework sequences are changed for those of the "acceptor" antibody.

An antibody can be of any the five major classes (isotypes) of immunoglobulins: IgA, IgD, IgE, IgG, and IgM, or subclasses thereof (e.g. IgG1, IgG2, IgG3, IgG4, lgA1 and IgA2), based on the identity of their heavy-chain constant domains referred to as alpha, delta, epsilon, gamma, and mu, respectively. The different classes of immunoglobulins have different and well known subunit structures and three-dimensional configurations. Antibodies can be naked or conjugated to other molecules such as therapeutic agents or diagnostic agents to form immunoconjugates.

The term "anticalin" refers to a protein that is derived from the lipocalin and that been engineered to bind to a specific target (see Skerra, 2008. FEBS J. 275(11):2677-83).

The term "antigen-binding fragment" or "Fab" refers to an antibody fragment comprising one constant and one variable domain of each of the heavy and light chain. A Fab fragment may be obtained by digesting an intact monoclonal antibody with papain.

The term "cancer" refers to a group of diseases, which can be defined as any abnormal benign or malignant new growth of tissue that possesses no physiological function and arises from uncontrolled usually rapid cellular proliferation and has the potential to invade or spread to other parts of the body.

The term "CD229" or "cluster of differentiation 229" refers to the T-lymphocyte surface antigen Ly-9, which is a protein that in humans is encoded by the LY9 gene. Exemplary sequence and data related to human CD229 has been deposited in the UniProtKB database under ID numbers Q9HBG7 and Q5VYI1. CD229 possesses four Ig-like domains, while the rest of SLAMF receptors contain two Ig-like domains.

"CD229-positive" cancer, including a "CD229-positive" cancerous disease, is one comprising cells, which have CD229 present at their cell surface. The term "CD229-positive" also refers to a cancer that produces sufficient levels of CD229 at the surface of cells thereof, such that a CAR-comprising cell of the present invention has a therapeutic effect, mediated by the binding of the CAR to CD229. In some embodiments, the CD229-positive cancer is multiple myeloma cancer.

The term "CD229-targeting moiety" refers to a substance that is able to bind CD229. Within the context of a CAR, a CD229-targeting moiety targets T cells to a CD229-positive cell, preferably a cancer cell. Within the context of a CAR, it is to be understood that the CD229-targeting moiety is genetically encodable.

The term "chimeric antigen receptor" or "CAR" refers to a synthetic receptor that targets T cells to a chosen antigen and reprograms T cell function, metabolism and persistence. Similarly, the term "CART" refers to a T cell that comprises a CAR.

"Combination therapy", "in combination with" or "in conjunction with" as used herein denotes any form of concurrent, parallel, simultaneous, sequential or intermittent treatment with at least two distinct treatment modalities (i.e., compounds, components, targeted agents or therapeutic agents). As such, the terms refer to administration of one treatment modality before, during, or after administration of the other treatment modality to the subject. The modalities in combination can be administered in any order. The therapeutically active modalities are administered together (e.g., simultaneously in the same or separate compositions, formulations or unit dosage forms) or separately (e.g., on the same day or on different days and in any order as according to an appropriate dosing protocol for the separate compositions, formulations or unit dosage forms) in a manner and dosing regimen prescribed by a medical care taker or according to a regulatory agency. In general, each treatment modality will be administered at a dose and/or on a time schedule determined for that treatment modality. Optionally, three or more modalities may be used in a combination therapy. Additionally, the combination therapies provided herein may be used in conjunction with other types of treatment. For example, other anti-cancer treatment may be selected from the group consisting of chemotherapy, surgery, radiotherapy (radiation) and/or hormone therapy, amongst other treatments associated with the current standard of care for the subject.

A "complete response" or "complete remission" or "CR" indicates the disappearance of all target lesions as defined in the RECIST v1.1 guideline. This does not always mean the cancer has been cured.

The term "costimulatory signaling domain" refers to a signaling moiety that provides to T cells a signal which, in addition to the primary signal provided by for instance the CD3ζ chain of the TCR/CD3 complex, mediates a T cell response, including, but not limited to, activation, proliferation, differentiation, cytokine secretion, and the like. A co-stimulatory domain can include all or a portion of, but is not limited to, CD27, CD28, 4-1BB (CD137), OX40 (CD134), CD30, CD40, 1COS, lymphocyte function-associated antigen-1 (LFA-1), CD2, CD7, LIGHT, NKG2C, B7-H3, and a ligand that specifically binds with CD83. In some embodiments, the co-stimulatory signaling domain is an intracellular signaling domain that interacts with other intracellular mediators to mediate a cell response including activation, proliferation, differentiation and cytokine secretion, and the like.

The term "designed ankyrin repeat proteins" or "DARPin" refers to a protein that is derived from an ankyrin repeat that has been engineered to bind to a specific target (see Plückthun, 2015. Annu Rev Pharmacol Toxicol. 55:489-511).

"Disease free survival" (DFS) refers to the length of time during and after treatment that the patient remains free of disease.

As used herein, the term "effective amount" of an agent, e.g., a therapeutic agent such as a CART, is that amount sufficient to effect beneficial or desired results, for example, clinical results, and, as such, an "effective amount" depends upon the context in which it is being applied. For example, in the context of administering a therapeutic agent that treats T-ALL, an effective amount can reduce the number of cancer cells; reduce the tumor size or burden; inhibit (i.e., slow to some extent and in a certain embodiment, stop) cancer cell infiltration into peripheral organs; inhibit (i.e., slow to some extent and in a certain embodiment, stop) tumor metastasis; inhibit, to some extent, tumor growth; relieve to some extent one or more of the symptoms associated with the cancer; and/or result in a favorable response such as increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof. The term "effective amount" can be used interchangeably with "effective dose," "therapeutically effective amount," or "therapeutically effective dose".

The term "fynomer" refers to a protein that is derived from the SH3 domain of human Fyn kinase that has been engineered to bind to a specific target (see Bertschinger et al., 2007. Protein Eng Des Sel. 20(2):57-68).

The terms "individual", "patient" or "subject" are used interchangeably in the present application to designate a human being and are not meant to be limiting in any way. The "individual", "patient" or "subject" can be of any age, sex and physical condition. The term "patient in need thereof" usually refers to a patient who suffers from a CD229-positive cancer.

"Infusion" or "infusing" refers to the introduction of a therapeutic agent-containing solution into the body through a vein for therapeutic purposes. Generally, this is achieved via an intravenous bag.

"Intracellular signaling domain" as used herein refers to all or a portion of one or more domains of a molecule (here the chimeric receptor molecule) that provides for activation of a lymphocyte. Intracellular domains of such molecules mediate a signal by interacting with cellular mediators to result in proliferation, differentiation, activation and other effector functions. Examples of intracellular signaling domains for use in a CAR of the invention include the intracellular sequences of the CD3ζ chain, and/or co-receptors that act in concert to initiate signal transduction following CAR engagement, as well as any derivative or variant of these sequences and any synthetic sequence that has the same functional capability. T cell activation can be said to be mediated by two distinct classes of cytoplasmic signaling sequence: those that initiate antigen-dependent primary activation and provide a T cell receptor like signal (primary cytoplasmic signaling sequences) and those that act in an antigen- independent manner to provide a secondary or co-stimulatory signal (secondary cytoplasmic signaling sequences). Primary cytoplasmic signaling sequences that act in a stimulatory manner may contain signaling motifs which are known as receptor tyrosine-based activation motifs or ITAMs. Examples of ITAM containing primary cytoplasmic signaling sequences include those derived from CD3ζ, FcRy, CD3γ, CD3δ, CD3ε, CD5, CD22, CD79a, CD79b, and CD66d.

The term "monobody" refers to a protein that is derived from a fibronectin type III domain that has been engineered to bind to a specific target (see Koide et al., 2013. J Mol Biol. 415(2):393-405).

The term "nanobody" refers to a protein comprising the soluble single antigen-binding V-domain of a heavy chain antibody, preferably a camelid heavy chain antibody (see Bannas et al., 2017. Front Immunol. 8:1603).

"Overall Survival" (OS) refers to the time from patient enrollment to death or censored at the date last known alive. OS includes a prolongation in life expectancy as compared to naive or untreated individuals or patients. Overall survival refers to the situation wherein a patient remains alive for a defined period of time, such as one year, five years, etc., e.g., from the time of diagnosis or treatment.

A "partial response" or "PR" refers to at least a 30% decrease in the sum of diameters of target lesions, taking as reference the baseline sum diameter, in response to treatment, as defined in the RECIST v1.1 guideline.

The term "peptide aptamer" refers to a short, 5-20 amino acid residue sequence that can bind to a specific target. Peptide aptamers are typically inserted within a loop region of a stable protein scaffold (see Reverdatto et al., 2015. Curr Top Med Chem. 15(12):1082-101).

As used herein, "pharmaceutically acceptable carrier" or "pharmaceutically acceptable diluent" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thiosulfate; low molecular weight proteins, such as human serum albumin, bovine serum albumin, gelatin, or other immunoglobulins; and hydrophilic polymers, such as polyvinylpyrrolidone. Other pharmaceutically acceptable carriers, excipients, or stabilizers, such as those described in Remington's Pharmaceutical Sciences 16th edition, Osol, A. Ed. (1980) may also be included in a pharmaceutical composition described herein, provided that they do not adversely affect the desired characteristics of the pharmaceutical composition.

"Progressive disease" or "disease that has progressed" refers to the appearance of one more new lesions or tumors and/or the unequivocal progression of existing non-target lesions as defined in the RECIST v1.1 guideline. Progressive disease or disease that has progressed can also refer to a tumor growth of more than 20 percent since treatment began, either due to an increase in mass or in spread of the tumor.

"Progression free survival" (PFS) refers to the time from enrollment to disease progression or death. PFS is generally measured using the Kaplan-Meier method and Response Evaluation Criteria in Solid Tumors (RECIST) 1.1 standards. Generally, progression free survival refers to the situation wherein a patient remains alive, without the cancer getting worse.

The term "RECIST" means Response Evaluation Criteria in Solid Tumours. RECIST guideline, criteria, or standard, describes a standard approach to solid tumor measurement and definitions for objective assessment of change in tumor size for use in adult and pediatric cancer clinical trials. RECIST v1.1 means version 1.1 of the revised RECIST guideline and it is published in European Journal of Cancers 45 (2009) 228-247.

The term "repebody" refers to a protein that is derived from a leucine-rich repeat module and that been engineered to bind to a specific target (see Lee et al., 2012. PNAS. 109(9): 3299-3304).

The term "respond favorably" generally refers to causing a beneficial state in a subject. With respect to cancer treatment, the term refers to providing a therapeutic effect on the subject. Positive therapeutic effects in cancer can be measured in a number of ways. For example, tumor growth inhibition, molecular marker expression, serum marker expression, and molecular imaging techniques can all be used to assess therapeutic efficacy of an anti-cancer therapeutic. A favorable response can be assessed, for example, by increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof.

The term "sequence identity" refers to a percentage value obtained when two sequences are compared using a pairwise sequence alignment tool. In the present case, the sequence identity is obtained using the global alignment tool "EMBOSS Needle" using the default settings (Rice et al., 2000. Trends Genet. 16(6):276-7; Li et al., 2015. Nucleic Acids Res. 43(W1):W580-4). The global alignment tool is available at: https://www.ebi.ac.uk/Tools/psa/ .

The term "single-chain antigen-binding fragment" or "scFab" refers to a fusion protein comprising one variable and one constant domain of the light chain of an antibody attached to one variable and one constant domain of the heavy chain of an antibody, wherein the heavy and light chains are linked together through a short peptide.

The term "single-chain variable fragment" or "scFv" refers to a fusion protein comprising the variable domains of the heavy chain and light chain of an antibody linked to one another with a peptide linker. The term also includes a disulfide stabilized Fv (dsFv).

"Stable disease" refers to disease without progression or relapse as defined in the RECIST v1.1 guideline. In stable disease there is neither sufficient tumor shrinkage to qualify for partial response, nor sufficient tumor increase to qualify as progressive disease.

"Time to Tumor Progression" (TTP) is defined as the time from enrollment to disease progression. TTP is generally measured using the RECIST v1.1 criteria.

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

### DESCRIPTION OF THE EMBODIMENTS

Four different CD229 CARs sequences (named GL001, GL002, GL005 and GL006) based on two different antibodies (hLy9 1.84 and hLy9 10.169) were generated and tested herein. GL001 and GL002 are based on hLy9 1.84 scFV (see Fig. 1) and share CDRs in both VL and VH chains, but the order of the VH and VL chain is inverted. On the other hand, GL005 and GL006 are based in hLy9 10.169, thereby also sharing their CDRs and also being different in the order of the VH and VL chains, see Fig. 1.

First, the expression of the newly developed CAR sequences in HEK293T cells and T was cells tested. As shown in Fig. 2, only GL001 and GL002 (VH-VL-41BB-CD3z and VL-VH-41BB-CD3z), based in hLy9 1.84 scFV, could be detected, while GL005 and GL006 (VH-VL-41BB-CD3z and VL-VH-41BB-CD3z) were not. In view of these results, the following tests were only performed with GL001 and GL002 CARS.

The efficacy of GL001 and GL002 cells was tested against myeloma cell lines by co-culturing T cells and myeloma cell lines. GL001 and GL002 CAR T cells conferred specific cytolytic activity against U266 and MM.1S myeloma cell lines with a dose-response relationship from high to low E:T ratios, indicating thar GL001 and GL002 CAR T cells exhibit a potent and specific cytotoxic effect against CD229-positive cells in vitro, see Fig. 3.

To better characterise GL001 and GL002 cell response upon CD229 binding, cell proliferation was measured. As shown in Figure 4, GL001 and GL002 cells proliferated in contact with the CD229+ MM.1S cell line and in response to interleukin-2 (IL-2). Some proliferation was observed in the absence of stimulus or in contact with a CD229-negative cell line (K562), confirming that cell proliferation was mediated by CD229 recognition, see Fig. 4.

Next, GL001 and GL002 cell production of cytokines was measured in the supernatant of effector-target cell co-cultures. It was observed that GL001 and GL002 cells showed a significant increase in IFNg and IL-2 pro-inflammatory cytokines when cocultured with CD229 positive cells (U266 and MM.1S), see Fig. 5

Lastly, to evaluate the efficacy of the GL001 and GL002 cells in vivo, a xenograft experiment in NOD.Cg-Prkdcscid II2rdtm1Wjl/SzJ (NSG) mice was performed. As shown in Figure 6, disease progression was clearly observed following U266 cell infusion with UTD cell group. The group that received U266 tumor cell line and GL002 demonstrated better disease control compared with UTD and GL001 groups. In terms of efficacy, these data suggest that GL002 is better than GL001.

To recapitulate human MM, the established BCMA heterogeneous disease was modelled creating a BCMA-knockout (KO) MM.1S myeloma cell line. GL001 and GL002 CAR T cells could lyse these BCMA-KO cells, see Fig. 7B. Further, as shown in Fig. 8, GL002 is superior to ARI002h, which is a CAR directed against BCMA antigen, used as a positive control. Importantly, these results show that GL002 is superior to anti-BCMA CARs in heterogeneous tumours, where a population of negative BCMA cells is present.

Preferably, the first aspect relates to a CD229 targeting moiety comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of [SSVSY] (SEQ ID NO: 4), or a sequence with at least 85%, 86%, 87%, 88%, 89%, 90%, 91%, 92%, 93%, 94%, 95%, 96%, 97%, 98%, 99% or 100% sequence identity to SEQ ID NO: 4;
- LCDR2 comprises, consists, or consists essentially of [DTS] (SEQ ID NO: 5), or a sequence with at least 85%, 86%, 87%, 88%, 89%, 90%, 91%, 92%, 93%, 94%, 95%, 96%, 97%, 98%, 99% or 100% sequence identity to SEQ ID NO: 5;
- LCDR3 comprises, consists, or consists essentially of [CQQWSSYPPTF] (SEQ ID NO: 6), or a sequence with at least 85%, 86%, 87%, 88%, 89%, 90%, 91%, 92%, 93%, 94%, 95%, 96%, 97%, 98%, 99% or 100% sequence identity to SEQ ID NO: 6;
- HCDR1 comprises, consists, or consists essentially of [GYTFTHYY] (SEQ ID NO: 7), or a sequence with at least 85%, 86%, 87%, 88%, 89%, 90%, 91%, 92%, 93%, 94%, 95%, 96%, 97%, 98%, 99% or 100% sequence identity to SEQ ID NO: 7;
- HCDR2 comprises, consists, or consists essentially of [IFPESGST] (SEQ ID NO: 8), or a sequence with at least 85%, 86%, 87%, 88%, 89%, 90%, 91%, 92%, 93%, 94%, 95%, 96%, 97%, 98%, 99% or 100% sequence identity to SEQ ID NO: 8; and
- HCDR3 comprises, consists, or consists essentially of [CARGTGFFDYW] (SEQ ID NO: 9), or a sequence with at least 85%, 86%, 87%, 88%, 89%, 90%, 91%, 92%, 93%, 94%, 95%, 96%, 97%, 98%, 99% or 100% sequence identity to SEQ ID NO: 9.

In some embodiments, the CD229-targeting moiety is an antibody, anticalin, repebody, monobody, scFv, Fab, scFab, affibody, fynomer, DARPin, nanobody, or peptide aptamer that specifically binds to CD229.

In some embodiments, the CD229-targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of [SSVSY] (SEQ ID NO: 4), LCDR2 consists of [DTS] (SEQ ID NO: 5), LCDR3 consists of [CQQWSSYPPTF] (SEQ ID NO: 6), HCDR1 consists of [GYTFTHYY] (SEQ ID NO: 7), HCDR2 consists of [IFPESGST] (SEQ ID NO: 8), and HCDR3 consists of [CARGTGFFDYW] (SEQ ID NO: 9).

Preferably, the CD229 targeting moiety is an antibody, F(ab')2, Fab, preferably scFv, or scFab comprising a VL domain and a VH domain, wherein the VL domain comprises, consists, or consists essentially of SEQ ID NO: 1, or a sequence with at least 85%, 86%, 87%, 88%, 89%, 90%, 91%, 92%, 93%, 94%, 95%, 96%, 97%, 98%, 99% or 100% sequence identity to SEQ ID NO: 1; and wherein the VH domain comprises, consists, or consists essentially of SEQ ID NO: 2 , or a sequence with at least 85%, 86%, 87%, 88%, 89%, 90%, 91%, 92%, 93%, 94%, 95%, 96%, 97%, 98%, 99% or 100% sequence identity to SEQ ID NO: 2. In some embodiments, the CD229-targeting moiety is an antibody, scFv, Fab, or scFab comprising a VL domain and VH domain, wherein the VL domain consists of SEQ ID NO: 1 and the VH domain consists of SEQ ID NO: 2.

The VL and VH of the CD229 targeting moiety described herein may be humanized. In the humanization process, the framework regions of the variable region of a targeting moiety may be modified, whereas the CDRs are maintained constant. Therefore, in some embodiments, the CD229 targeting moiety is an antibody, F(ab')2, Fab, preferably scFv, or scFab, comprising a VL and a VH domains, wherein the VL domain comprises a sequence having at least 90%, 91%, 92%, 93%, 94%, 95%, 96%, 97%, 98%, 99% or 100% sequence identity to SEQ ID NO: 1, wherein the CDRs comprised in said VL are maintained constant and consists of SEQ ID NOs: 4, 5, and 6; and wherein the VH domain comprises a sequence having at least 90%, 91%, 92%, 93%, 94%, 95%, 96%, 97%, 98%, 99% or 100% sequence identity to SEQ ID NO: 2, wherein the CDRs comprised in said VH are maintained constant and consists of SEQ ID NOs: 7, 8, and 9.

Thus, in an embodiment, the CD229 targeting moiety of the invention comprises a VL domain and a VH domain, wherein
- said VL domain comprises a sequence with at least 80%, 85%, 86%, 87%, 88%, 89%, 90%, 91%, 92%, 93%, 94%, 95%, 96%, 97%, 98%, or 99% sequence identity to SEQ ID NO: 1, and wherein said VL domain comprises CDR regions consisting of LCDR1 [SSVSY] (SEQ ID NO: 4), LCDR2 [DTS] (SEQ ID NO: 5), LCDR3 [CQQWSSYPPTF] (SEQ ID NO: 6), and
- said VH domain comprises a sequence with at least 80%, 85%, 86%, 87%, 88%, 89%, 90%, 91%, 92%, 93%, 94%, 95%, 96%, 97%, 98%, or 99% sequence identity to SEQ ID NO: 2 , and wherein said VH domain comprises CDR regions consisting of HCDR1 [GYTFTHYY] (SEQ ID NO: 7), HCDR2 [IFPESGST] (SEQ ID NO: 8), and HCDR3 [CARGTGFFDYW] (SEQ ID NO: 9), and preferably, wherein the CD229 targeting moiety is capable of binding to human CD229, preferably as measured by surface plasmon resonance (SPR).

In an embodiment, the CD229 targeting moiety of the invention is capable of binding to human CD229, wherein the CD229 targeting moiety comprises a VL and a VH domain, wherein:
- the VL domain comprises a sequence having at least 90%, 91%, 92%, 93%, 94%, 95%, 96%, 97%, 98%, 99% or 100% sequence identity to SEQ ID NO: 1, and
- the VH domain comprising a sequence having at least 90%, 91%, 92%, 93%, 94%, 95%, 96%, 97%, 98%, 99% or 100% sequence identity to SEQ ID NO: 2, and
wherein the LCDR1, 2, and 3 comprised in the VL domain consist of SEQ ID NO: 4, 5 and 6, respectively, and wherein the HCDR1, 2 and 3 comprised in said VH domain consists of SEQ ID NO: 7, 8 and 9, respectively.

By "capable of binding to human CD229" is meant that the CD229 targeting moiety has binding affinity, for human CD229. Preferably, the binding affinity is specific, i.e., the CD229 is capable of specifically binding to the human CD229 (which is its target molecule) in a manner which distinguishes from the binding to non-target molecules, i.e., from other molecules that are not CD229. Thus, the CD229 targeting moiety either does not bind to non-target molecules, or exhibits negligible or substantially-reduced (as compared to the target) e. g. background, binding to nontarget molecules. The CD229 targeting moiety specifically recognises CD229. Specific binding between the CD229 targeting moiety and the human CD229 can be assessed by numerous techniques, such as surface plasmon resonance, flow cytometry, or ELISA assays.

In a preferred embodiment, the VL and the VH domains comprised in the CD229-targeting moiety are linked by a peptide linker. In some embodiments, the linker comprises at least 5 amino acids, preferably between 5 and 25, preferably between 10-20 amino acids, most preferably 20 amino acids. Preferably, the amino acid is G and/or S. Preferably, the peptide linker comprises, consists or consists essentially of SEQ ID NO: 14, or a sequence that has 85%, 86%, 87%, 88%, 89%, 90%, 91%, 92%, 93%, 94%, 95%, 96%, 97%, 98%, 99% or 100% sequence identity to SEQ ID NO: 14. Preferably, the amino acid is G and/or S. Preferably, the peptide linker comprises, consists or consists essentially of SEQ ID NO: 16, or a sequence that has 85%, 86%, 87%, 88%, 89%, 90%, 91%, 92%, 93%, 94%, 95%, 96%, 97%, 98%, 99% or 100% sequence identity to SEQ ID NO: 16. Preferably, the VL and VH of GL001 (SEQ ID NOs: 1 and 2) are linked with the peptide linker of SEQ ID NO: 14, as depicted in SEQ ID NO: 3. Preferably, the VL and VH of GL002 (SEQ ID NOs: 1 and 2) are linked with the peptide linker of SEQ ID NO: 16, as depicted in SEQ ID NO: 17.

In a preferred embodiment, the CD229-targeting moiety further comprises a signal peptide that is placed preferably at the N-terminal region of the CD229-targeting moiety. Preferably, the signal peptide comprises, consists or consists essentially of SEQ ID NO: 15, or a sequence that has 85%, 86%, 87%, 88%, 89%, 90%, 91%, 92%, 93%, 94%, 95%, 96%, 97%, 98%, 99% or 100% sequence identity to SEQ ID NO: 15.

Preferably, the CD229 targeting moiety comprises, consists, or consists essentially of SEQ ID NO: 3 (GL001 Signal peptide +VH+Linker+VL), or a sequence that has 85%, 86%, 87%, 88%, 89%, 90%, 91%, 92%, 93%, 94%, 95%, 96%, 97%, 98%, 99% or 100% sequence identity to SEQ ID NO: 3. Most preferably, the CD229 targeting moiety consists of SEQ ID NO: 3 (GL001 Signal peptide +VH+Linker+VL).

Preferably, the CD229 targeting moiety comprises, consists, or consists essentially of SEQ ID NO: 17 (GL002 Signal peptide +VL+Linker+VH), or a sequence that has 85%, 86%, 87%, 88%, 89%, 90%, 91%, 92%, 93%, 94%, 95%, 96%, 97%, 98%, 99% or 100% sequence identity to SEQ ID NO: 17. Most preferably, the CD229 targeting moiety consists of SEQ ID NO: 17 (GL002 Signal peptide +VL+Linker+VH).

In some embodiments, the CD229 targeting moiety is a scFv comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of [SSVSY] (SEQ ID NO: 4), or a sequence with at least 85%, 86%, 87%, 88%, 89%, 90%, 91%, 92%, 93%, 94%, 95%, 96%, 97%, 98%, 99% or 100% sequence identity to SEQ ID NO: 4;
- LCDR2 comprises, consists, or consists essentially of [DTS] (SEQ ID NO: 5), or a sequence with at least 85%, 86%, 87%, 88%, 89%, 90%, 91%, 92%, 93%, 94%, 95%, 96%, 97%, 98%, 99% or 100% sequence identity to SEQ ID NO: 5;
- LCDR3 comprises, consists, or consists essentially of [CQQWSSYPPTF] (SEQ ID NO: 6), or a sequence with at least 85%, 86%, 87%, 88%, 89%, 90%, 91%, 92%, 93%, 94%, 95%, 96%, 97%, 98%, 99% or 100% sequence identity to SEQ ID NO: 6;
- HCDR1 comprises, consists, or consists essentially of [GYTFTHYY] (SEQ ID NO: 7), or a sequence with at least 85%, 86%, 87%, 88%, 89%, 90%, 91%, 92%, 93%, 94%, 95%, 96%, 97%, 98%, 99% sequence identity to SEQ ID NO: 7;
- HCDR2 comprises, consists, or consists essentially of [IFPESGST] (SEQ ID NO: 8), or a sequence with at least 85%, 86%, 87%, 88%, 89%, 90%, 91%, 92%, 93%, 94%, 95%, 96%, 97%, 98%, 99% or 100% sequence identity to SEQ ID NO: 8; and
- HCDR3 comprises, consists, or consists essentially of [CARGTGFFDYW] (SEQ ID NO: 9), or a sequence with at least 85%, 86%, 87%, 88%, 89%, 90%, 91%, 92%, 93%, 94%, 95%, 96%, 97%, 98%, 99% or 100% sequence identity to SEQ ID NO: 9.

In some embodiments, the CD229-targeting moiety is a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of [SSVSY] (SEQ ID NO: 4), LCDR2 consists of [DTS] (SEQ ID NO: 5), LCDR3 consists of [CQQWSSYPPTF] (SEQ ID NO: 6), HCDR1 consists of [GYTFTHYY] (SEQ ID NO: 7), HCDR2 consists of [IFPESGST] (SEQ ID NO: 8), and HCDR3 consists of [CARGTGFFDYW] (SEQ ID NO: 9).

Preferably, the CD229 targeting moiety is a scFv comprising a VL domain and a VH domain, wherein the VL domain comprises, consists, or consists essentially of SEQ ID NO: 1, or a sequence with at least 85%, 86%, 87%, 88%, 89%, 90%, 91%, 92%, 93%, 94%, 95%, 96%, 97%, 98%, 99% or 100% sequence identity to SEQ ID NO: 1; and wherein the VH domain comprises, consists, or consists essentially of SEQ ID NO: 2, or a sequence with at least 85%, 86%, 87%, 88%, 89%, 90%, 91%, 92%, 93%, 94%, 95%, 96%, 97%, 98%, 99% or 100% sequence identity to SEQ ID NO: 2. In some embodiments, the CD229-targeting moiety is a scFv comprising a VL domain and VH domain, wherein the VL domain consists of SEQ ID NO: 1 and the VH domain consists of SEQ ID NO: 2.

Preferably, the CD229 targeting moiety is a scFv that comprises, consists, or consists essentially of SEQ ID NO: 3 (GL001 Signal peptide +VH+Linker+VL), or a sequence that has 85%, 86%, 87%, 88%, 89%, 90%, 91%, 92%, 93%, 94%, 95%, 96%, 97%, 98%, 99% or 100% sequence identity to SEQ ID NO: 3. Most preferably, the CD229 targeting moiety is a scFv consists of SEQ ID NO: 3 (GL001 Signal peptide +VH+Linker+VL).

Preferably, the CD229 targeting moiety is a scFv that comprises, consists, or consists essentially of SEQ ID NO: 17 (GL002 Signal peptide +VL+Linker+VH), or a sequence that has 85%, 86%, 87%, 88%, 89%, 90%, 91%, 92%, 93%, 94%, 95%, 96%, 97%, 98%, 99% or 100% sequence identity to SEQ ID NO: 17. Most preferably, the CD229 targeting moiety is a scFv consists of SEQ ID NO: 17 (GL002 Signal peptide +VL+Linker+VH).

The CD229 targeting moieties as defined above can be part of a chimeric antigen receptor. Thus, in a preferred embodiment of the first aspect, the present invention provides a chimeric antigen receptor (CAR) comprising:
a) an extracellular domain comprising a CD229 targeting moiety as defined in the first aspect or in any of the embodiments disclosed above,
b) a transmembrane domain; and
c) an intracellular signaling domain.

Each of the elements of the CAR according to this embodiment of the first aspect are further developed below:
*a)* *extracellular domain comprising a CD229 targeting moiety* as *defined in the first aspect or any of its embodiments.*

In some embodiments, the CD229-targeting moiety is a scFv comprising a VL domain and VH domain, wherein said VL domain comprises LCDR1, LCDR2 and LCDR3 polypeptides and said VH domain comprises HCDR1, HCDR2 and HCDR3 polypeptides, and wherein
- LCDR1 comprises, consists, or consists essentially of [SSVSY] (SEQ ID NO: 4), or a sequence with at least 85%, 86%, 87%, 88%, 89%, 90%, 91%, 92%, 93%, 94%, 95%, 96%, 97%, 98%, 99% or 100% sequence identity to SEQ ID NO: 4;
- LCDR2 comprises, consists, or consists essentially of [DTS] (SEQ ID NO: 5), or a sequence with at least 85%, 86%, 87%, 88%, 89%, 90%, 91%, 92%, 93%, 94%, 95%, 96%, 97%, 98%, 99% or 100% sequence identity to SEQ ID NO: 5;
- LCDR3 comprises, consists, or consists essentially of [CQQWSSYPPTF] (SEQ ID NO: 6), or a sequence with at least 85%, 86%, 87%, 88%, 89%, 90%, 91%, 92%, 93%, 94%, 95%, 96%, 97%, 98%, 99% or 100% sequence identity to SEQ ID NO: 6;
- HCDR1 comprises, consists, or consists essentially of [GYTFTHYY] (SEQ ID NO: 7), or a sequence with at least 85%, 86%, 87%, 88%, 89%, 90%, 91%, 92%, 93%, 94%, 95%, 96%, 97%, 98%, 99% or 100% sequence identity to SEQ ID NO: 7;
- HCDR2 comprises, consists, or consists essentially of [IFPESGST] (SEQ ID NO: 8), or a sequence with at least 85%, 86%, 87%, 88%, 89%, 90%, 91%, 92%, 93%, 94%, 95%, 96%, 97%, 98%, 99% or 100% sequence identity to SEQ ID NO: 8; and
- HCDR3 comprises, consists, or consists essentially of [CARGTGFFDYW] (SEQ ID NO: 9), or a sequence with at least 85%, 86%, 87%, 88%, 89%, 90%, 91%, 92%, 93%, 94%, 95%, 96%, 97%, 98%, 99% or 100% sequence identity to SEQ ID NO: 9.

In some embodiments, the CD229-targeting moiety is a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 4, LCDR2 consists of SEQ ID NO: 5, LCDR3 consists of SEQ ID NO: 6, HCDR1 consists of SEQ ID NO: 7, HCDR2 consists of SEQ ID NO: 8, and HCDR3 consists of SEQ ID NO: 9.

In an embodiment of the first aspect, the extracellular domain comprising a CD229-targeting moiety is a scFv comprising a VL domain and a VH domain, wherein the VL domain comprises, consists, or consists essentially of SEQ ID NO: 1, or a sequence that has 85%, 86%, 87%, 88%, 89%, 90%, 91%, 92%, 93%, 94%, 95%, 96%, 97%, 98%, or 100% sequence identity to SEQ ID NO: 1; and wherein the VH domain comprises, consists, or consists essentially of SEQ ID NO: 2, or a sequence that has 85%, 86%, 87%, 88%, 89%, 90%, 91%, 92%, 93%, 94%, 95%, 96%, 97%, 98%, 99% or 100% sequence identity to SEQ ID NO: 2.

Most preferably, the CD229 targeting moiety is a scFv that comprises, consists, or consists essentially of SEQ ID NO: 3, or a sequence that has 85%, 86%, 87%, 88%, 89%, 90%, 91%, 92%, 93%, 94%, 95%, 96%, 97%, 98%, 99% or 100% sequence identity to SEQ ID NO: 3 (GL001 Signal peptide+VH+linker+VL). Most preferably, the CD229 targeting moiety is a scFV that consists of SEQ ID NO: 3.

Most preferably, the CD229 targeting moiety is a scFv that comprises, consists, or consists essentially of SEQ ID NO: 17, or a sequence that has 85%, 86%, 87%, 88%, 89%, 90%, 91%, 92%, 93%, 94%, 95%, 96%, 97%, 98%, 99% or 100% sequence identity to SEQ ID NO: 17 (GL002 Signal peptide+VH+linker+VL). Most preferably, the CD229 targeting moiety is a scFV that consists of SEQ ID NO: 17.

In an embodiment, the CD229 targeting moiety of the invention is capable of binding to human CD229, wherein the CD229 targeting moiety is a scFv and comprises a VL and a VH domain, wherein:
- the VL domain comprises a sequence having at least 90%, 91%, 92%, 93%, 94%, 95%, 96%, 97%, 98%, 99% or 100% sequence identity to SEQ ID NO: 1, and
- the VH domain comprising a sequence having at least 90%, 91%, 92%, 93%, 94%, 95%, 96%, 97%, 98%, 99% or 100% sequence identity to SEQ ID NO: 2, and
wherein the LCDR1, 2, and 3 comprised in the VL domain consist of SEQ ID NO: 4, 5 and 6, respectively, and wherein the HCDR1, 2 and 3 comprised in said VH domain consists of SEQ ID NO: 7, 8 and 9, respectively.

In a preferred embodiment, the VL and the VH domains comprised in the scFv are linked by a peptide linker. Preferably, the peptide linker comprises, consists or consists essentially of SEQ ID NO: 14 or 16, or a sequence that has 85%, 86%, 87%, 88%, 89%, 90%, 91%, 92%, 93%, 94%, 95%, 96%, 97%, 98%, 99% or 100% sequence identity to SEQ ID NO: 14 or 16. Preferably, the VL and VH of the scFV GL001 are linked by a peptide linker that comprises, consists or consists essentially of SEQ ID NO: 14, or a sequence that has 85%, 86%, 87%, 88%, 89%, 90%, 91%, 92%, 93%, 94%, 95%, 96%, 97%, 98%, 99% or 100% sequence identity to SEQ ID NO: 14. Preferably, the VL and VH of the scFV GL002 are linked by a peptide linker that comprises, consists or consists essentially of SEQ ID NO: 16, or a sequence that has 85%, 86%, 87%, 88%, 89%, 90%, 91%, 92%, 93%, 94%, 95%, 96%, 97%, 98%, 99% or 100% sequence identity to SEQ ID NO: 16.

In a preferred embodiment, the CD229-targeting moiety is a scFv and it further comprises a signal peptide that is placed at the N-terminal region of the CD229-targeting moiety. Preferably, the signal peptide comprises, consists or consists essentially of SEQ ID NO: 15, or a sequence that has 85%, 86%, 87%, 88%, 89%, 90%, 91%, 92%, 93%, 94%, 95%, 96%, 97%, 98%, 99% or 100% sequence identity to SEQ ID NO: 15.

### b) transmembrane domain

The transmembrane domain may be derived either from a natural or a synthetic source. When the source is natural, the domain may be derived from any membrane-bound or transmembrane protein. Transmembrane regions may comprise at least the transmembrane region(s) of the α-, β- or ζ- chain of CD28, CD3, CD45, CD4, CD8, CD9, CD16, CD22, CD33, CD37, CD64, CD80, CD86, CD134, CD137, or CD154.

A transmembrane domain may be synthetic or a variant of a naturally occurring transmembrane domain. In some embodiments, synthetic or variant transmembrane domains comprise predominantly hydrophobic residues such as leucine and valine.

In some embodiments, the transmembrane domain comprises the transmembrane domain of CD28, CD3, CD45, CD4, CD8, CD9, CD16, CD22, CD33, CD37, CD64, CD80, CD86, CD134, CD137, CD154, or a variant thereof, wherein the variant thereof has a 95% sequence identity.

In some embodiments, the transmembrane domain comprises the transmembrane domain of CD28, CD3, CD45, CD4, CD8, CD9, CD16, CD22, CD33, CD37, CD64, CD80, CD86, CD134, CD137, CD154, or a variant thereof, wherein the variant thereof has a 98% sequence identity.

In some embodiments, the transmembrane domain comprises the transmembrane domain of CD28, CD3, CD45, CD4, CD8, CD9, CD16, CD22, CD33, CD37, CD64, CD80, CD86, CD134, CD137, or CD154.

In some embodiments, the transmembrane domain comprises the transmembrane domain of CD8 or a variant thereof, wherein the variant thereof has a 95% sequence identity.

In some embodiments, the transmembrane domain comprises the transmembrane domain of CD8 or a variant thereof. Preferably, the transmembrane domain consists or consists essentially of SEQ ID NO: 10, or a sequence that has 85%, 86%, 87%, 88%, 89%, 90%, 91%, 92%, 93%, 94%, 95%, 96%, 97%, 98%, 99% or 100% sequence identity to SEQ ID NO: 10.

In an embodiment, the transmembrane domain is linked to a hinge domain, wherein the hinge domain is preferably placed at the N-terminal of the transmembrane domain. Preferably, the hinge domain consists or consists essentially of SEQ ID NO: 18, or a sequence that has 85%, 86%, 87%, 88%, 89%, 90%, 91%, 92%, 93%, 94%, 95%, 96%, 97%, 98%, 99% or 100% sequence identity to SEQ ID NO: 18.

Therefore, in a preferred embodiment, the transmembrane domain linked to a hinge domain consists or consists essentially of SEQ ID NO: 19, or a sequence that has 85%, 86%, 87%, 88%, 89% 90%, 91%, 92%, 93%, 94%, 95%, 96%, 97%, 98%, 99% or 100% sequence identity to SEQ ID NO: 19.

### c) Intracellular signaling domain

The intracellular signaling domain provides for the activation of at least one function of the cell expressing the CAR upon binding to the ligand expressed on tumor cells. In some embodiments, the intracellular signaling domain contains one or more intracellular signaling domains. In some embodiments, the intracellular signaling domain is a portion of and/or a variant of an intracellular signaling domain that provides for activation of at least one function of the CAR-comprising cell.

In some embodiments, the intracellular signaling domain comprises the intracellular domain of CD3ζ, FcRy, CD3γ, CD3δ, CD3ε, CD5, CD22, CD79a, CD79b, CD66b, or a variant thereof, wherein the variant thereof has a 95% sequence identity. In some embodiments, the intracellular signaling domain comprises the intracellular domain of CD3ζ, FcRy, CD3γ, CD3δ, CD3ε, CD5, CD22, CD79a, CD79b, CD66b, or a variant thereof, wherein the variant thereof has a 98% sequence identity. In some embodiments, the intracellular signaling domain comprises the intracellular domain of CD3ζ, FcRy, CD3γ, CD3δ, CD3ε, CD5, CD22, CD79a, CD79b or CD66b.

In some embodiments, the intracellular signaling domain comprises the intracellular domain of CD3ζ or a variant thereof, wherein the variant thereof has a 95% sequence identity. In some embodiments, the intracellular signaling domain comprises the intracellular domain of CD3ζ or a variant thereof, wherein the variant thereof has a 98% sequence identity. In some embodiments, the intracellular signaling domain comprises the intracellular domain of CD3ζ.

In a preferred embodiment, the intracellular signalling domain consists or consists essentially of SEQ ID NO: 11, or a sequence that has 85%, 86%, 87%, 88%, 89% 90%, 91%, 92%, 93%, 94%, 95%, 96%, 97%, 98%, 99% or 100% sequence identity to SEQ ID NO: 11.

In some embodiments, the intracellular signaling domain comprises SEQ ID NO: 11 or a sequence that has 95% sequence identity to SEQ ID NO: 11. In some embodiments, the intracellular signaling domain comprises SEQ ID NO: 11 or a sequence that has 98% sequence identity to SEQ ID NO: 11. In some embodiments, the intracellular signaling domain comprises SEQ ID NO: 11 or a sequence that has 99% sequence identity to SEQ ID NO: 11. In some embodiments, the intracellular signaling domain comprises SEQ ID NO: 11. In some embodiments, the intracellular signaling domain consists of SEQ ID NO: 11.

Optionally, at least a costimulatory signaling domain may also be present in the CAR according to the first aspect or any of its embodiments:

### d) Costimulatory signaling domain

In some embodiments, the CAR may further comprise a costimulatory signaling domain. In some embodiments, the costimulatory signaling domain comprises the intracellular domain of CD27, CD28, CD137, CD134, CD30, CD40, lymphocyte function-associated antigen-1 (LFA-1), CD2, CD7, LIGHT, NKG2C, CD276 or a variant thereof, wherein the variant thereof has a 95% sequence identity.

In some embodiments, the costimulatory signaling domain comprises the intracellular domain of CD27, CD28, CD137, CD134, CD30, CD40, lymphocyte function-associated antigen-1 (LFA-1), CD2, CD7, LIGHT, NKG2C, CD276 or a variant thereof, wherein the variant thereof has a 98% sequence identity.

In some embodiments, the costimulatory signaling domain comprises the intracellular domain of CD27, CD28, CD137 or 4-1BB, CD134, CD30, CD40, lymphocyte function-associated antigen-1 (LFA-1), CD2, CD7, LIGHT, NKG2C, or CD276.

In some embodiments, the costimulatory signaling domain comprises the intracellular domain of CD137 or a variant thereof, wherein the variant thereof has a 95% sequence identity. In some embodiments, the costimulatory signaling domain comprises the intracellular domain of CD137 or 4-1BB or a variant thereof, wherein the variant thereof has a 98% sequence identity. In some embodiments, the costimulatory signaling domain comprises the intracellular domain of CD137 or 4-1BB.

In some embodiments, the costimulatory signaling domain comprises SEQ ID NO: 12 or a sequence that has 95% sequence identity to SEQ ID NO: 12.In some embodiments, the costimulatory signaling domain comprises SEQ ID NO: 12 or a sequence that has 98% sequence identity to SEQ ID NO: 12. In some embodiments, the costimulatory signaling domain comprises SEQ ID NO: 12 or a sequence that has 99% sequence identity to SEQ ID NO: 12. In some embodiments, the costimulatory signaling domain comprises SEQ ID NO: 12. In some embodiments, the costimulatory signaling domain consists of SEQ ID NO: 12.

In a preferred embodiment, the costimulatory signaling domain consists or consists essentially of SEQ ID NO: 12, or a sequence that has 85%, 86%, 87%, 88%, 89% 90%, 91%, 92%, 93%, 94%, 95%, 96%, 97%, 98%, 99% or 100% sequence identity to SEQ ID NO: 12.

### Full sequence CARs according to the first aspect of the present invention

In some embodiments, the CAR comprises:
(i) a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 4, LCDR2 consists of SEQ ID NO: 5, LCDR3 consists of SEQ ID NO: 6, HCDR1 consists of SEQ ID NO: 7, HCDR2 consists of SEQ ID NO: 8, and HCDR3 consists of SEQ ID NO: 9;
(ii) a transmembrane domain comprising SEQ ID NO: 19 or a sequence that has 95% sequence identity to SEQ ID NO: 19;
(iii) an intracellular signaling domain comprising SEQ ID NO: 11 or a sequence that has 95% sequence identity to SEQ ID NO: 11; and
(iv) a costimulatory signaling domain comprising SEQ ID NO: 12 or a sequence that has 95% sequence identity to SEQ ID NO: 12.

In some embodiments, the CAR comprises:
(i) a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 4, LCDR2 consists of SEQ ID NO: 5, LCDR3 consists of SEQ ID NO: 6, HCDR1 consists of SEQ ID NO: 7, HCDR2 consists of SEQ ID NO: 8, and HCDR3 consists of SEQ ID NO: 9;
(ii) a transmembrane domain comprising SEQ ID NO: 19 or a sequence that has 98% sequence identity to SEQ ID NO: 19;
(iii) an intracellular signaling domain comprising SEQ ID NO: 11 or a sequence that has 98% sequence identity to SEQ ID NO: 11; and
(iv) a costimulatory signaling domain comprising SEQ ID NO: 12 or a sequence that has 98% sequence identity to SEQ ID NO: 12.

In some embodiments, the CAR comprises:
(i) a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 4, LCDR2 consists of SEQ ID NO: 5, LCDR3 consists of SEQ ID NO: 6, HCDR1 consists of SEQ ID NO: 7, HCDR2 consists SEQ ID NO: 8, and HCDR3 consists of SEQ ID NO: 9;
(ii) a transmembrane domain comprising SEQ ID NO: 19 or a sequence that has 98% sequence identity to SEQ ID NO: 19;
(iii) an intracellular signaling domain comprising SEQ ID NO: 11 or a sequence that has 99% sequence identity to SEQ ID NO: 11; and
(iv) a costimulatory signaling domain comprising SEQ ID NO: 12 or a sequence that has 99% sequence identity to SEQ ID NO: 12.

In some embodiments, the CAR comprises:
(i) a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 4, LCDR2 consists of SEQ ID NO: 5, LCDR3 consists of SEQ ID NO: 6, HCDR1 consists of SEQ ID NO: 7, HCDR2 consists SEQ ID NO: 8, and HCDR3 consists of SEQ ID NO: 9;
(ii) a transmembrane domain comprising SEQ ID NO: 19;
(iii) an intracellular signaling domain comprising SEQ ID NO: 11; and
(iv) a costimulatory signaling domain comprising SEQ ID NO: 12.

In some embodiments, the CAR comprises:
(i) a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 4, LCDR2 consists of SEQ ID NO: 5, LCDR3 consists of SEQ ID NO: 6, HCDR1 consists of SEQ ID NO: 7, HCDR2 consists of SEQ ID NO: 8, and HCDR3 consists of SEQ ID NO: 9;
(ii) a transmembrane domain consisting of SEQ ID NO: 19;
(iii) an intracellular signaling domain consisting of SEQ ID NO: 11; and
(iv) a costimulatory signaling domain consisting of SEQ ID NO: 12.

In some embodiments, the CAR comprises:
(i) a scFv comprising a VL domain and VH domain, wherein the VL domain consists of SEQ ID NO: 1 and the VH domain consists of SEQ ID NO: 2;
(ii) a transmembrane domain comprising SEQ ID NO: 19 or a sequence that has 95% sequence identity to SEQ ID NO: 19;
(iii) an intracellular signaling domain comprising SEQ ID NO: 11 or a sequence that has 95% sequence identity to SEQ ID NO: 11; and
(iv) a costimulatory signaling domain comprising SEQ ID NO: 12 or a sequence that has 95% sequence identity to SEQ ID NO: 12.

In some embodiments, the CAR comprises:
(i) a scFv comprising a VL domain and VH domain, wherein the VL domain consists of SEQ ID NO: 1 and the VH domain consists of SEQ ID NO: 2;
(ii) a transmembrane domain comprising SEQ ID NO: 19 or a sequence that has 98% sequence identity to SEQ ID NO: 19;
(iii) an intracellular signaling domain comprising SEQ ID NO: 11 or a sequence that has 98% sequence identity to SEQ ID NO: 11; and
(iv) a costimulatory signaling domain comprising SEQ ID NO: 12 or a sequence that has 98% sequence identity to SEQ ID NO: 12.

In some embodiments, the CAR comprises:
(i) a scFv comprising a VL domain and VH domain, wherein the VL domain consists of SEQ ID NO: 1 and the VH domain consists of SEQ ID NO: 2;
(ii) a transmembrane domain comprising SEQ ID NO: 19 or a sequence that has 98% sequence identity to SEQ ID NO: 19;
(iii) an intracellular signaling domain comprising SEQ ID NO: 11 or a sequence that has 99% sequence identity to SEQ ID NO: 11; and
(iv) a costimulatory signaling domain comprising SEQ ID NO: 12 or a sequence that has 99% sequence identity to SEQ ID NO: 12.

In some embodiments, the CAR comprises:
(i) a scFv comprising a VL domain and VH domain, wherein the VL domain consists of SEQ ID NO: 1 and the VH domain consists of SEQ ID NO: 2;
(ii) a transmembrane domain comprising SEQ ID NO: 19;
(iii) an intracellular signaling domain comprising SEQ ID NO: 11; and
(iv) a costimulatory signaling domain comprising SEQ ID NO: 12.

In some embodiments, the CAR comprises:
(i) a scFv comprising a VL domain and VH domain, wherein the VL domain consists of SEQ ID NO: 1 and the VH domain consists of SEQ ID NO: 2;
(ii) a transmembrane domain consisting of SEQ ID NO: 19;
(iii) an intracellular signaling domain consisting of SEQ ID NO: 11; and
(iv) a costimulatory signaling domain consisting of SEQ ID NO: 12.

In some embodiments, the CAR comprises:
(i) a scFv consisting of SEQ ID NOs: 3 or 17;
(ii) a transmembrane domain comprising SEQ ID NO: 19 or a sequence that has 95% sequence identity to SEQ ID NO: 19;
(iii) an intracellular signaling domain comprising SEQ ID NO: 11 or a sequence that has 95% sequence identity to SEQ ID NO: 11; and
(iv) a costimulatory signaling domain comprising SEQ ID NO: 12 or a sequence that has 95% sequence identity to SEQ ID NO: 12.

In some embodiments, the CAR comprises:
(i) a scFv consisting of SEQ ID NOs: 3 or 17;
(ii) a transmembrane domain comprising SEQ ID NO: 19 or a sequence that has 98% sequence identity to SEQ ID NO: 19;
(iii) an intracellular signaling domain comprising SEQ ID NO: 11 or a sequence that has 98% sequence identity to SEQ ID NO: 11; and
(iv) a costimulatory signaling domain comprising SEQ ID NO: 12 or a sequence that has 98% sequence identity to SEQ ID NO: 12.

In some embodiments, the CAR comprises:
(i) a scFv consisting of SEQ ID NOs: 3 or 17;
(ii) a transmembrane domain comprising SEQ ID NO: 19 or a sequence that has 98% sequence identity to SEQ ID NO: 19;
(iii) an intracellular signaling domain comprising SEQ ID NO: 11 or a sequence that has 99% sequence identity to SEQ ID NO: 11; and
(iv) a costimulatory signaling domain comprising SEQ ID NO: 12 or a sequence that has 99% sequence identity to SEQ ID NO: 12.

In some embodiments, the CAR comprises:
(i) a scFv consisting of SEQ ID NOs: 3 or 17;
(ii) a transmembrane domain comprising SEQ ID NO: 19;
(iii) an intracellular signaling domain comprising SEQ ID NO: 11; and
(iv) a costimulatory signaling domain comprising SEQ ID NO: 12.

In some embodiments, the CAR comprises:
(i) a scFv consisting of SEQ ID NOs: 3 or 17;
(ii) a transmembrane domain consisting of SEQ ID NO: 19;
(iii) an intracellular signaling domain consisting of SEQ ID NO: 11; and
(iv) a costimulatory signaling domain consisting of SEQ ID NO: 12.

In some embodiments, the CAR comprises or consists of SEQ ID NO: 13 or a sequence that has 95% sequence identity with SEQ ID NO: 13. In some embodiments, the CAR comprises or consists of SEQ ID NO: 13 or a sequence that has 98% sequence identity with SEQ ID NO: 13. In some embodiments, the CAR comprises or consists of SEQ ID NO: 13 or a sequence that has 99% sequence identity with SEQ ID NO: 13. In some embodiments, the CAR comprises or consists of SEQ ID NO: 13.

In some embodiments, the CAR comprises or consists of SEQ ID NO: 18 or a sequence that has 95% sequence identity with SEQ ID NO: 18. In some embodiments, the CAR comprises or consists of SEQ ID NO: 18 or a sequence that has 98% sequence identity with SEQ ID NO: 18. In some embodiments, the CAR comprises or consists of SEQ ID NO: 18 or a sequence that has 99% sequence identity with SEQ ID NO: 18. In some embodiments, the CAR comprises or consists of SEQ ID NO: 18.

### Nucleic acid

In **a second aspect,** the present invention provides a nucleic acid encoding any one of the CD229 targeting moiety of the present invention, including any one of the CARs disclosed above. The nucleic acid sequence that encodes the chimeric receptor links together a number of modular components that can be excised and replaced with other components in order to customize the chimeric receptor for efficient T cell activation and recognition of CD229.

In some embodiments, the nucleic acid is suitable for transducing or transforming a cell. In some embodiments, the nucleic acid is suitable for transducing or transforming a T cell for use in adoptive immunotherapy.

In some embodiments, the nucleic acid is codon optimized for expression in mammalian cells. Codon optimization methods are known in the art.

The nucleic acid of the present invention may be comprised in a γ-retroviral or lentiviral vector which can be used to transduce or transform a T cell. The nucleic acid may also be inserted into a cell through the use of DNA transposons, RNA transfection or genome editing techniques such as TALEN, ZFN and CRISPR/Cas9.

### Cells

In a **third aspect,** the present invention provides a cell comprising the nucleic acid of the present invention and/or the CAR of the present invention. In some embodiments, the cell is a T-cell (referred to as a CART).

In some embodiments, the cell is a naive T cell, memory stem T cell or central memory T cell. It is currently thought that these cells are better suited for adaptive immunotherapy.

In some embodiments, the cell is an autologous T cell. The term "autologous cell" refers to a cell obtained from the same patient that is to be treated using any one of the methods of the present invention.

In some embodiments, the cell is an allo-tolerant T cell. The term "allo-tolerant cell" refers to a cell that has been engineered to decrease the risk of a Graft-versus-host disease response. In some embodiments, this is achieved by genomic editing-mediated deletion of TCR and/or β2-microglobulin. Allo-tolerant cells are known in the art.

In some embodiments, the T cell is a CD3-positive T cell. In some embodiments, the T cell is T a CD8+ T cell. In some instances, they can be a human cell.

In some embodiments, the cell is a lymphoid precursor, embryonic stem cell or an induced pluripotent stem cell with the capacity to differentiate into a mature T cell.

### Pharmaceutical composition

In **a fourth aspect,** the present invention provides a pharmaceutical composition comprising a plurality of cells of the present invention and a pharmaceutically acceptable carrier or diluent.

A pharmaceutical composition as described herein may also contain other substances. These substances include, but are not limited to, cryoprotectants, surfactants, antioxidants, and stabilizing agents. The term "cryoprotectant" as used herein, includes agents which provide stability to the CARTs against freezing-induced stresses. Nonlimiting examples of cryoprotectants include sugars, such as sucrose, glucose, trehalose, mannitol, mannose, and lactose; polymers, such as dextran, hydroxyethyl starch and polyethylene glycol; surfactants, such as polysorbates (e.g., PS-20 or PS-80); and amino acids, such as glycine, arginine, leucine, and serine. A cryoprotectant exhibiting low toxicity in biological systems is generally used.

In some embodiments, the cells are formulated by first harvesting them from their culture medium, and then washing and concentrating the cells in a medium and container system suitable for administration (a "pharmaceutically acceptable" carrier) in a therapeutically effective amount. Suitable infusion medium can be any isotonic medium formulation, typically normal saline, Normosol R (Abbott) or Plasma-Lyte A (Baxter), but also 5% dextrose in water or Ringer's lactate can be utilized. The infusion medium can be supplemented with human serum albumin, fetal bovine serum or other human serum components.

### Methods of treatment

In a **fifth aspect,** the present invention provides a CD229 targeting moiety according to the first aspect, a nucleic acid according to the second aspect, a cell according to the third aspect, and/or a pharmaceutical composition according to the fourth aspect, for use as a medicament or in therapy. In a preferred embodiment, the use is in a method of treating a CD229-positive cancer comprising administering the CD229 targeting moiety of the first aspect, the nucleic acid of the second aspect, the cell of the third aspect and/or the pharmaceutical composition of the fourth aspect to a patient in need thereof. In some embodiments, the CD229-positive cancer is multiple myeloma (MM) cancer. In an embodiment, the multiple myeloma is selected from the list consisting of Light Chain Myeloma, Non-secretory Myeloma, Solitary Plasmacytoma, Extramedullary Plasmacytoma, Monoclonal Gammopathy of Undetermined Significance (MGUS), Smoldering Multiple Myeloma (SMM), Immunoglobulin D (IgD) Myeloma, Immunoglobulin E (IgE) Myeloma. In a preferred embodiment, the MM cancer comprises heterogeneous tumours that include cells negative for BCMA receptor. Therefore, in a preferred embodiment, the cell or the composition of the present invention is used to treat a BCMA negative tumour.

In an embodiment, the CD229-positive cancer is a lymphoma, preferably selected from the list consisting of chronic lymphocytic leukemia (CLL), classic mantle-cell lymphoma (MCL), follicular lymphoma (FL), and/or diffuse large B-cell lymphoma (DLBCL), or any combination thereof. Preferably, the CD229-positive cancer is a B-cell lineage lymphoma, preferably a marginal-zone lymphomas (MZL), mucosa-associated lymphoid tissue lymphoma (MALT), and splenic marginal-zone lymphoma (SMZL).

In some embodiments, the CD229 targeting moiety, nucleic acid, cell or pharmaceutical composition is administered intravenously, intraperitoneally, into the bone marrow, into the lymph node, and /or into cerebrospinal fluid.

Thus, disclosed are methods of treating multiple myeloma comprising administering an effective amount of a CD229 targeting moiety, a nucleic acid, cell (preferably T cell) genetically modified to express one or more of the disclosed CAR polypeptides, or pharmaceutical composition, to a subject in need thereof. For example, disclosed are methods of treating multiple myeloma comprising administering an effective amount of a T cell genetically modified to express a CAR polypeptide comprising a CD229 antigen binding domain, a hinge and transmembrane domain, and an intracellular signaling domain, as described above.

In some embodiments, the method comprises a combination therapy. Thus, the CD229 targeting moiety and/or the CAR-expressing cell described herein may be used in combination with other known agents and therapies. Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

In an embodiment, the CD229 targeting moiety and/or the CAR-expressing cell described herein is administered with at least one additional therapeutic agent simultaneously, in the same or in separate compositions, or sequentially. For sequential administration, the CD229 targeting moiety and/or the CAR-expressing cell described herein can be administered first, and the additional agent can be administered second, or the order of administration can be reversed.

In an embodiment, the combination therapy comprises administration of at least two different CAR-expressing cells. In one embodiment, a CAR-expressing cell, e.g., an anti-BCMA CAR-expressing cell may be used in combination with the CAR-expressing cell described herein. In one embodiment, the subject in needs thereof is treated with a CAR anti-BCMA, and subsequently or simultaneously treated with the CAR of the present invention which is directed to target human CD229. This combination therapy is especially advantageous in tumours that comprise BCMA negative cells and thus are prone to relapse after anti-BCMA therapy.

In some embodiments, the CD229 positive cancer is relapsed/refractory multiple myeloma. In general, the relapse of MM can manifest several months or years after the initial remission; however, most relapses occur within two years after the initial treatment. Refractoriness is a term that implies that the patient has no longer responded to at least one therapy strategy after a relapse.

In some embodiments, the patient to be treated with the method of the present invention is in complete or near-complete remission after treatment with another therapy. It may be preferable desirable to decrease the tumor burden before using the methods of the present invention because since there are several alternative effector T-cells in cases of patients with highly active relapsed/refractory MM. In some embodiments, the patient to be treated with the method of the present invention has previously been treated with another therapy which resulted in a partial response, complete response, stable disease, decrease in progressive disease, reduced time to tumor progression or any combination thereof.

In some embodiments, the method comprises further administering an immune checkpoint. In a further embodiment, the method comprises further administering an immune checkpoint inhibitor and/or an IAP inhibitor. In some embodiments, the cell or pharmaceutical composition as described herein is administered in combination with chemotherapeutic agents and/or immunosuppressants. In an embodiment, a patient is first treated with a chemotherapeutic agent that inhibits or destroys other immune cells followed by the cell or pharmaceutical composition described herein. In some cases, chemotherapy may be avoided entirely. In some embodiments, the therapeutic agent can be, but is not limited to, conventional chemotherapy including but not limited to alkylating agents, antimetabolites, anti -microtubule agents, topoisomerase inhibitors, and cytotoxic antibiotics; high- dose chemotherapy including but not limited to high-dose Melphalan chemotherapy with or without stem cell transplant; proteasome inhibitors such as, but not limited to, bortezomib, ixazomib, and carfilzomib; immunomodulatory agents (IMiDS) such as, but not limited to, thalidomide, lenalidomide, and pomalidomide; histone deacetylase (HDAC) inhibitors such as, but not limited to panobinostat; monoclonal antibodies such as, but not limited to, daratumumab, isatuximab, or elotuzumab; bispecific antibodies; and immune checkpoint inhibitors such as, but not limited to, ipilimumab, nivolumab, and pembrolizumab.

### Kits

In a **sixth aspect,** the present invention provides kits and their use according to any cof the previous aspects. The materials described above as well as other materials can be packaged together in any suitable combination as a kit useful for performing, or aiding in the performance of, the disclosed method. It is useful if the kit components in a given kit are designed and adapted for use together in the disclosed method. Also disclosed are kits comprising one or more of the vectors disclosed herein.

### SEQUENCE LISTING

### Common sequences in GL001 and GL002 antibodies:

### VL CDRs:

- **LCDR1: SEQ ID NO: 4:** SSVSY
- **LCDR2: SEQ ID NO: 5:** DTS
- **LCDR3: SEQ ID NO: 6:** CQQWSSYPPTF

### VH CDRs:

- **HCDR1: SEQ ID NO: 7** GYTFTHYY
- **HCDR2: SEQ ID NO: 8** IFPESGST
- **HCDR3: SEQ ID NO: 9** CARGTGFFDYW

**SEQ ID NO: 1: VL** 
QIVLTQSPAIMSASPGEKVTVTCSASSSVSYMFWYQQKPGFSPRLLIYDTSNLASGVP
VRFSGSGSGTSYSLTISRMEAEDAATYYCQQWSSYPPTFGGGTKLEIK
**SEQ ID NO: 2: VH** 
QMQLQESGPELVKPGASVKISCKASGYTFTHYYINWVQQRPGQGLEWIGWIFPESGS
TYYSEKFKAKATLTVDKSSSTAYMLLSSLTSEDSAVYFCARGTGFFDYWGQGTTLTV
SS
**SEQ ID NO: 15: Signal Peptide**
   MEAPAQLLFLLLLWLPDTTG
**SEQ ID NO: 18: CD8 hinge**
   TTTPAPRPPTPAPTIASQPLSLRPEACRPAAGGAVHTRGLDFACDI
**SEQ ID NO: 10: CD8 TM**
   YIWAPLAGTCGVLLLSLVITLYC
**SEQ ID NO: 19: CD8 hinge + TM** 
TTTPAPRPPTPAPTIASQPLSLRPEACRPAAGGAVHTRGLDFACDIYIWAPLAGTCGV
LLLSLVITLYC
**SEQ ID NO: 12: 4-1BB**
   KRGRKKLLYIFKQPFMRPVQTTQEEDGCSCRFPEEEEGGCEL
**SEQ ID NO: 11: CD3z**

RVKFSRSADAPAYQQGQNQLYNELNLGRREEYDVLDKRRGRDPEMGGKPQRRKNP
QEGLYNELQKDKMAEAYSEIGMKGERRRGKGHDGLYQGLSTATKDTYDALHMQALP
PR

### Sequences of Scfv GL001 mVH VL CD229.84 BB CAR

**SEQ ID NO: 14: Linker (G4S)x3**
   GGGGSGGGGSGGGGS
**SEQ ID NO: 3: Signal peptide + VH + linker + VL** 
MEAPAQLLFLLLLWLPDTTGQMQLQESGPELVKPGASVKISCKASGYTFTHYYINWV
QQRPGQGLEWIGWIFPESGSTYYSEKFKAKATLTVDKSSSTAYMLLSSLTSEDSAVYF
CARGTGFFDYWGQGTTLTVSSGGGGSGGGGSGGGGSQIVLTQSPAIMSASPGEKV
 
TVTCSASSSVSYMFWYQQKPGFSPRLLIYDTSNLASGVPVRFSGSGSGTSYSLTISR
MEAEDAATYYCQQWSSYPPTFGGGTKLEIK
**SEQ ID NO: 13: Signal peptide + VH + linker + VL + CD8 hinge + CD8 TM + 4-1BB + CD3z**

MEAPAQLLFLLLLWLPDTTGQMQLQESGPELVKPGASVKISCKASGYTFTHYYINWV
QQRPGQGLEWIGWIFPESGSTYYSEKFKAKATLTVDKSSSTAYMLLSSLTSEDSAVYF
CARGTGFFDYWGQGTTLTVSSGGGGSGGGGSGGGGSQIVLTQSPAIMSASPGEKV
TVTCSASSSVSYMFWYQQKPGFSPRLLIYDTSNLASGVPVRFSGSGSGTSYSLTISR
MEAEDAATYYCQQWSSYPPTFGGGTKLEIKTTTPAPRPPTPAPTIASQPLSLRPEACR
PAAGGAVHTRGLDFACDIYIWAPLAGTCGVLLLSLVITLYCKRGRKKLLYIFKQPFMRP
VQTTQEEDGCSCRFPEEEEGGCELRVKFSRSADAPAYQQGQNQLYNELNLGRREEY
DVLDKRRGRDPEMGGKPQRRKNPQEGLYNELQKDKMAEAYSEIGMKGERRRGKGH
DGLYQGLSTATKDTYDALHMQALPPR

### Sequences of pGL002 mVL VH CD299.84 BB CAR

**SEQ ID NO: 16: Linker (G4S)x4**
   GGGGSGGGGSGGGGSGGGGS
**SEQ ID NO: 17: Signal peptide + VL + linker + VH** 
MEAPAQLLFLLLLWLPDTTGQIVLTQSPAIMSASPGEKVTVTCSASSSVSYMFWYQQK
PGFSPRLLIYDTSNLASGVPVRFSGSGSGTSYSLTISRMEAEDAATYYCQQWSSYPP
TFGGGTKLEIKGGGGSGGGGSGGGGSGGGGSQMQLQESGPELVKPGASVKISCKA
SGYTFTHYYINWVQQRPGQGLEWIGWIFPESGSTYYSEKFKAKATLTVDKSSSTAYM
LLSSLTSEDSAVYFCARGTGFFDYWGQGTTLTVSS
**SEQ ID NO: 18: Signal peptide + VL + linker + VH+ CD8 hinge + CD8 TM + 4-1BB + CD3z**

MEAPAQLLFLLLLWLPDTTGQIVLTQSPAIMSASPGEKVTVTCSASSSVSYMFWYQQK
PGFSPRLLIYDTSNLASGVPVRFSGSGSGTSYSLTISRMEAEDAATYYCQQWSSYPP
TFGGGTKLEIKGGGGSGGGGSGGGGSGGGGSQMQLQESGPELVKPGASVKISCKA
SGYTFTHYYINWVQQRPGQGLEWIGWIFPESGSTYYSEKFKAKATLTVDKSSSTAYM
LLSSLTSEDSAVYFCARGTGFFDYWGQGTTLTVSSTTTPAPRPPTPAPTIASQPLSLR
PEACRPAAGGAVHTRGLDFACDIYIWAPLAGTCGVLLLSLVITLYCKRGRKKLLYIFKQ
PFMRPVQTTQEEDGCSCRFPEEEEGGCELRVKFSRSADAPAYQQGQNQLYNELNL
GRREEYDVLDKRRGRDPEMGGKPQRRKNPQEGLYNELQKDKMAEAYSEIGMKGER
RRGKGHDGLYQGLSTATKDTYDALHMQALPPR

The invention is described below by the following examples, which must be considered as merely illustrative and in no case limiting of the scope of the present invention.

### EXAMPLES

### EXAMPLE 1:

### Materials and Methods - CAR Design

### Production of anti-Human Ly9 antibodies

Anti- Ly9 (CD229 mAbs were generated by fusing NS-1 myeloma cells with spleen cells from BALB/c mice immunized three times with 30×10⁶ cells of the mouse pre-B-cell line 300.19, stably transfected with the full-length cDNA (clone 13). The hybridomas were subclones twice. The antibodies were further validated using cell lines and peripheral blood leukocytes.

Clone Hly9.1.84 was first described in paper: de la Fuente MA, Tovar V, Villamor N, Zapater N, Pizcueta P, Campo E, Bosch J, Engel P. Molecular characterization and expression of a novel human leukocyte cell-surface marker homologous to mouse Ly-9. Blood. 2001 Jun 1;97(11):3513-20.

The mapping to the second domain of antibody HLy91.184 was published in paper: Romero X, Zapater N, Calvo M, Kalko SG, de la Fuente MA, Tovar V, Ockeloen C, Pizcueta P, Engel P. CD229 (Ly9) lymphocyte cell surface receptor interacts homophilically through its N-terminal domain and relocalizes to the immunological synapse. J Immunol. 2005 Jun 1;174(11):7033-42.

The mapping to the first or second domain was done as described in Romero et al 2005. Three different human Ly9 constructs were used: full-length human Ly9, a Ly9 isoform that contains only domain 1 and a chimeric mouse human molecule where the second Ig domain of Ly9 was substituted the corresponding mouse domain. The transfected cells were stained with different anti-human antibodies using flow cytometry.

**Table II. Mapping the interaction domain of CD229 mAbs^{a}**

| **mAbs** | **CD229 hDI/DII mDIII/DIV** | **CD229 hDI/ DIII/DIV mDII** | **CD229 ADI** | **mAb Interaction Domain*^{b}*** |
|---|---|---|---|---|
| **HLy9.1.25** | + | + | - | **DI** |
| **HLy9.1.38** | + | + | - | **DI** |
| **HLy9.1.77** | + | + | - | **DI** |
| **HLy9.1.84** | + | - | + | **OIl** |

| | | | | |
|---|---|---|---|---|
| *^{a}* COS cells were transiently transfected with chimeric human (h)/mouse (m) constructs or the isoform lacking the first V-like Ig domain (CD229 ΔDI) described in *Materials and Methods.* CD229 mabs were assayed by flow cytometry: +, Detection of protein expression, -, nondetection of protein expression. *^{b}* Domain recognized by the mAb. | | | | |

### Cloning, plasmid, and lentiviral production:

We designed four CD229 CARs sequences with a CD8α hinge and transmembrane domain, a 4-1BB costimulatory, and CD3z signaling domains. The sequences of the heavy (HV) and light variable (LV) regions of each antibody (hLy9 1.84 and hLy9 10.169) were used to generate a total of 4 single-chain variable fragments (scFv) domains. A linker with 15 or 20 amino acids was added between the VH-VL or VL-VH sequences, respectively.
- HLy9 1.84
   ∘ HLy9 1.84 VH-VL-41BB-CD3z also called GL001
   ∘ HLy9 1.84 VL-VH-41BB-CD3z also called GL002
- HLy9 10.169
   ∘ HLy9 10.169 VH-VL-41BB-CD3z also called GL005
   ∘ HLy9 10.169 VL-VH-41BB-CD3z also called GL006

The complete CAR sequences (including single peptide, scFv, CD8 hinge, and transmembrane regions 4-1BB and CD3z) were synthesized by GenScript and cloned into the third-generation lentiviral vector pCCL, under the control of EF1a promoter.

To produce lentiviral particles for pre-clinical studies, HEK293T cells were transfected with our transfer vectors (pCCL-EF1α-CARCD229) together with packaging plasmids pMDLg-pRRE (Addgene, 12251), pRSV-Rev (Addgene, 12253), and envelope plasmid pMD2.G (Addgene, 12259), using linear PEI molecular weight (MW) 25,000 (Polysciences, 23966-1). Briefly, 5 × 10⁶ HEK293T cells were plated 24 hr before transfection in 10-cm dishes. At the time of transfection, 14 µg total DNA (6.9 µg transfer vector, 1.7 µg pMDLg/pRRE, 3.41 µg pRSV-Rev, and 2 µg pMD2.G) was diluted in serum-free DMEM. 35 µg PEI was added to the mix and incubated for 20 min at room temperature. After incubation, DNA-PEI complexes were added to the cells cultured in 7 mL complete DMEM. Viral supernatants were collected 48 hr later and concentrated with LentiX-Concentrator (Clontech, Takara) following the manufacturer's protocol. Concentrated lentivirus was stored at -80°C until use.

### Lentivirus Titration

The number of transducing units (TU/mL) was determined by the limiting dilution method. Briefly, HEK293T cells were seeded 24 hr before transduction. Then, 1:10 dilutions of the viral supernatant were prepared and added on top of the cells in complete DMEM + 5 µg/mL Polybrene. Cells were trypsinized 48 hr later and labeled with allophycocyanin (APC)-conjugated AffiniPureF(ab')₂ Fragment Goat anti-mouse immunoglobulin G (IgG) (Jackson ImmunoResearch Laboratories, 115-136-072) before being analyzed by flow cytometry. A dilution corresponding to 2%-20% of positive cells was used to calculate viral titer.

### Generation of CAR-expressing primary human T cells

PBMCs were isolated from healthy donor whole blood obtained from the BST (Blood and Tissue Bank of Barcelona) using Ficoll density gradient separation. T cells were subsequently isolated (negative isolation) from PBMCs using magnetism-activated cell sorting. Isolated T cells were stimulated with CD3/CD28 T-cell activation Dynabeads. In initial screens, T cells were lentivirally transduced 48h post stimulation. After that, Dynabeads were removed 7 days post stimulation. All T cells were expanded in complete T-cell medium, 5% AB human serum (Sigma, H4522), penicillin-streptomycin (100 µg/mL), and fed with IL-2 every 2-3 days. The expression of the CAR on the T cell surface was analyzed by flow cytometry. Experiments were performed after 8-10 days of T-cell expansion.

### Cell-line generation and maintenance

Multiple myeloma (MM) cell lines (U266, MM.1S, OPM2, and RPMI8226), and K562 cell line were purchased from American Tissue Culture Collection (ATCC, Manassas, VA). Cell lines K562, RPMI8226, and MM.1S were cultured in RPMI with 10% heat-inactivated Fetal Bovine Serum (FBS) and 1% penicillin-streptomycin and U266 with 15% FBS. HEK293-T cells were cultured with DMEM with 10% FBS and 1% penicillin-streptomycin. Tumor cell lines were lentivirally transduced to express the EGFP-firefly luciferase (Ffluc) fusion gene, and EGFP+ cells will be enriched by fluorescence-activated cell sorting (FACS) to >98% purity. BCMAneg tumor cells (BCMAKO) were generated by CRISPR/Cas9-mediated gene knockout. Tumor cells were nucleofected with ribonucleoprotein, consisting of chemically synthesized gRNA targeting BCMA complexed to purified Cas9 protein, using Electroporation Solution and the Nucleofector 2B Device (Lonza). The cells were subsequently bulk-sorted for BCMAneg populations by FACS.

### Multi-parameter flow cytometry:

To analyze CAR expression, cells were labelled with a recombinant Fc-tagged biotinylated CD229 protein (G&P Biosciences), and recombinant human CD229 His (R&D Systems) followed by anti-Fc, Streptavidin (BD Biosciences) or anti-His tag antibodies (Miltenyi Biotec). To evaluate T-cell phenotype and CRISPR/Cas9-mediated gene disruption of surface proteins, CAR-T cells were stained with the following antibodies: anti-CD3, anti-CD4, anti-CD8, anti-PD-1 (BD Biosciences); anti-TIGIT, anti-LAG-3, anti-TIM-3, anti-CCR7, anti-CD45RA, anti-CXCR3, anti-CD25, anti-CD71, anti-CD69, anti-CD28 and anti-CD27 (BioLegend). Tumor cells were stained with the following antibodies: anti-BCMA, anti-CD229, anti-CD38, and anti-CD138 (BioLegend). Live cells were gated by either forward/side scatter or negative uptake of a dye that stains dead cells such as propidium iodide or LIVE/Dead Aqua (ThermoFisher Scientific). For each sample, at least 50,000 events will be acquired on a cytometer BD FACSCanto II (BD Biosciences). FCS files will be analyzed with FlowJo Software v.10 (BD Biosciences).

**Functional assays:** *in vitro* assays will be done challenging CAR T-cells with MM cell lines (U266, MM.1S, OPM2, RPMI-8226 lines, and knock-out cell lines).

*Cytotoxicity assay:* The cytolytic activity was assessed in a luminescence-based assay. 5×10³ ffluc-transduced target cells were co-cultured with CAR T-cells at defined effector-to-target (E:T) ratios for 4 to 24 hours. Just prior to analysis, 0.15 mg/mL D-luciferin firefly (Biosynth) was added to the co-culture and luminescence was measured on an Infinite 200 Pro instrument (Tecan). Specific lysis was calculated using the standard formula.

*Proliferation assay:* T cells were labeled with 0.2 mmol/L carboxyfluorescein succinimidyl ester (CFSE; Invitrogen), washed, and plated in triplicate with stimulator cells in medium without exogenous cytokines. After 72-hour incubation, CART cells were labeled and analysed by flow cytometry to assess cell division.

*Cytokine production:* Cytokine secretion was analyzed in supernatant obtained from 24-hour co-cultures of T-cells with target cells at an E:T ratio of 4:1 (5×10⁴ T-cells) using commercially available ELISA kits (BioLegend).

*Safety evaluation, fratricide:* CD229 will be evaluated for fratricide with T-cells as well with peripheral blood cells from the same donor, in order to evaluate the activity against normal blood cells.

*In vivo xenograft studies in NSG mice:* All *in vivo* experiments were approved by the University of Barcelona Animal Research Committee. Eight- to twelve-week-old male and female NOD/SCID IL-2Rcnull (NSG) mice were used. MM cells (U266 and MM.1S cell lines) genetically modified to express GFP-Ffluc will be inoculated IV to the mice. This modification allows the follow-up of the disease by bioluminescence with D-luciferin.

Mice were irradiated at 2Gy at day-1. At day 0, mice received i.v. 1-5 ×10⁶ GFP-ffLuc-tumor cells. On day 14, mice were randomly allocated to receive i.v. either untransduced T cells or CART cells. Bioluminescence images were taken once a week to follow disease progression. In vivo imaging was recorded and analyzed with a Perkin Elmer IVIS Spectrum In Vivo Imaging System.

### Statistical analyses

Statistical analyses were conducted using Prism Software (GraphPad). Student t test was conducted as a two-sided paired test with a confidence interval of 95% and results with a P value less than 0.05 were considered significant. Statistical analyses of survival were conducted by log-rank testing and results with a P value less than 0.05 were considered significant.

### Results

### In Vitro Evaluation of different anti-CD229 CAR T-cells

The scFv of anti-CD229 CARs (HLy9 1.84 VH-VL-41BB-CD3z, HLy9 1.84 VL-VH-41BB-CD3z, HLy9 10.169 VH-VL-41BB-CD3z, and HLy9 10.169 VL-VH-41BB-CD3z) was cloned in frame with the rest of the CAR-signaling domains in a lentiviral vector (pCCL) (Figure 1A). For the evaluation of CARs efficacy, peripheral blood mononuclear cells (PBMCs) isolated from buffy-coats were activated using CD3 and CD28 dynabeads and subsequently transduced using anti-CD229 containing lentivirus.

### Lentiviral transduction / CAR expression on T cells

After the lentiviral infection and expansion period, the expression of anti-CD229 CARs on HEK293T cells and T cells was confirmed by flow cytometry only in hLy9 1.84 CARs (VH-VL-41BB-CD3z [GL001] and VL-VH-41BB-CD3z [GL002]) (Figure 2A), meanwhile, the CARs manufactured with hLy9 10.169 sequence (VH-VL-41BB-CD3z and VL-VH-41BB-CD3z), the anti-CD229 CAR was not detected (Figure 2B).

### Killing capacity of anti-CD229 CAR T cells

The efficacy of GL001 and GL002 cells was tested against myeloma cell lines by co-culturing T cells and myeloma cell lines. Cytotoxicity of GL001 and GL002 cells was measured by the in vitro eradication of the CD229 positive tumor cell lines (U266 and MM.1S). The K562 cell line was used as a negative control since it does not express CD229 on its surface. As a positive control, we used an effective CAR T-cell (ARI002h) developed at our institution (Perez-Amill, Haematologica, 2021), that is redirected against a well-known antigen expressed in myeloma, that is B-cell maturation antigen (BCMA). Both U266 and MM.1S cell lines express BCMA on its surface. The K562 tumor cell line does not express BCMA on its surface.

We confirmed that GL001 and GL002 CAR T cells conferred specific cytolytic activity against U266 and MM.1S myeloma cell lines with a dose-response relationship from high to low E:T ratios. We used untransduced (UTD) T cells as a reference and ARI002h (anti-BCMA) CAR T cells as comparators for specific target cell lysis (Figure 3).

All these data indicate that GL001 and GL002 CAR T cells exhibit a potent and specific cytotoxic effect against CD229-positive cells in vitro.

To better characterise GL001 and GL002 cell response upon CD229 binding, cell proliferation was measured by using a standard carboxyfluorescein diacetate succinimidyl ester (CFSE) assay at the 72-hr time point. Antigen binding should be able to promote GL001 and GL002 cell expansion in order to eliminate a tumor in vivo. As shown in Figure 4, GL001 and GL002 cells proliferated in contact with the CD229+ MM.1S cell line and in response to interleukin-2 (IL-2) (positive control). Some proliferation was observed in the absence of stimulus or in contact with a CD229-negative cell line (K562), confirming that cell proliferation was mediated by CD229 recognition.

GL001 and GL002 cell production of cytokines was measured in the supernatant of effector-target cell co-cultures after 24 hr and analyzed using an ELISA. Cytokine levels from co-cultures using GL001, GL002, UTD were compared (Figure 5). While UTD cells did not show an increase in IFNg and IL-2, GL001 and GL002 cells showed a significant increase in these two pro-inflammatory cytokines when cocultured with CD229 positive cells (U266 and MM.1S). There was not a significant increase in cytokines production in the absence of stimulus or in contact with CD229-negative cells (K562).

### In Vivo Evaluation of GL001 and GL002 Efficacy

To evaluate the efficacy of the GL001 and GL002 cells in vivo, we performed a xenograft experiment in NOD.Cg-Prkdc^{scid} II2rd^{tm1Wjl}/SzJ (NSG) mice.

Mice were randomly allocated to the administration of U266 plus UTD cells (A), U266 plus GL001 (B), U266 plus GL002 (C), and U266 plus ARI002h (D). Mice were inoculated with U266-FfLuc + GFP + (CD229+ / BCMA+) cells through their tail vein on day 1. On day 14, mice were infused with either UTD, GL001, GL002, or ARI002h cells.

As shown in Figure 6, disease progression was clearly observed following U266 cell infusion with UTD cell group. The group that received U266 tumor cell line and GL002 demonstrated better disease control compared with UTD and GL001 groups. However, there was better control of the disease and survival advantage in the group that received ARI002h compared with all the remaining groups. In terms of efficacy, these data suggest that GL002 is better than GL001.

To recapitulate human MM, we modelled the established BCMA heterogeneous disease. A CRISPR/Cas9-mediated BCMA-knockout (KO) MM.1S myeloma cell line was generated, with BCMA KO confirmed by flow cytometry and resistance to BCMA-targeted CAR T-cell cytotoxicity (ARI002h) Figure 7A. GL001 and GL002 CAR T cells could lyse these MM1S BCMA-KO cells Figure 7B.

We wanted to analyze the efficacy of GL002 against ARI002h at a lower dose in a model of established tumor including BCMA-negative disease. To develop the model, we intravenously injected mice with a mixture of 15% MM.1S BCMA-KO tumor cells and 85% of wild-type (WT) tumor cells with endogenous BCMA and CD229 expression. Mice were treated with subtherapeutic moderate (1 × 10⁶) doses of CAR T cells. As expected, in control UTD T-cell-treated mice, tumors progressed. In the group that received ARI002h, there was a progressive disease after a two-week control of myeloma. Nevertheless, there was a survival advantage in the group that received GL002. Figure 8. These data suggest that GL002 is superior to ARI002h when a population of negative BCMA tumor cells is present.

Finally, MM1.S myeloma cells recovered from the bone marrow of mice at the time of euthanasia showed that the tumor cells were BCMA⁺ (UTD group), BCMA⁻ (ARI002h group) and BCMA⁺ (GL2 or CD229-VLVH-BBz group), see Figure 9.

## Claims

1. A CD229 targeting moiety, wherein the CD229 targeting moiety is an antibody, F(ab')2, Fab, scFab or scFv, comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 consists of SEQ ID NO: 4 [SSVSY],
- LCDR2 consists of SEQ ID NO: 5 [DTS],
- LCDR3 consists of SEQ ID NO: 6 [CQQWSSYPPTF],
- HCDR1 consists of SEQ ID NO: 7 [GYTFTHYY],
- HCDR2 consists of SEQ ID NO: 8 [IFPESGST], and
- HCDR3 consists of SEQ ID NO: 9 [CARGTGFFDYWJ.

2. The CD229 targeting-moiety of claim 1, wherein
- the VL domain comprises a sequence with at least 85% sequence identity to SEQ ID NO: 1, and wherein said VL domain comprises CDR regions consisting of LCDR1 as set forth in SEQ ID NO: 4, LCDR2 as set forth in SEQ ID NO: 5, LCDR3 as set forth in SEQ ID NO: 6, and wherein
- the VH domain comprises a sequence with at least 85% sequence identity to SEQ ID NO: 2 , and wherein said VH domain comprises CDR regions consisting of HCDR1 as set forth in SEQ ID NO: 7, HCDR2 as set forth in SEQ ID NO: 8, and HCDR3 as set forth in SEQ ID NO: 9.

3. The CD229 targeting-moiety of claims 1 or 2, wherein the CD229 targeting-moiety is an antibody, F(ab')2, Fab, scFab or scFv, comprising a VL domain and VH domain, wherein the VL domain consists of SEQ ID NO: 1 and the VH domain consists of SEQ ID NO: 2.

4. The CD229 targeting-moiety of any of claims 1 to 3, wherein the CD229 targeting-moiety is a scFv.

5. The CD229 targeting-moiety of any of claims 1 to 4, wherein the CD229 targeting-moiety is a scFv consisting of SEQ ID NO: 17.

6. A chimeric antigen receptor (CAR) comprising:
a. an extracellular domain comprising a CD229 targeting-moiety, wherein the CD229 targeting-moiety is as defined in any of claims 1 to 5;
b. a transmembrane domain; and
c. an intracellular signaling domain.

7. The CAR according to claim 6, wherein the transmembrane domain comprises the transmembrane domain of CD8.

8. The CAR according to any of claims 6 or 7, wherein the intracellular signaling domain comprises the intracellular domain of CD3ζ.

9. The CAR according to any of claims 6 to 8, wherein the costimulatory signaling domain comprises the intracellular domain of CD137.

10. The CAR according to any one of claims 6 to 9, consisting of SEQ ID NO: 18.

11. A nucleic acid encoding the CAR according to any one of claims 6 to 10.

12. A cell comprising the nucleic acid according to claim 11.

13. The CD229 targeting-moiety according to any of claims 1 to 5, the CAR according to any of claims 6 to 10, the nucleic acid according to claim 11, or the cell according to claim 12, for use in a method of treating a CD229-positive cancer, wherein the method comprises administering the CD229 targeting moiety, the CAR, the nucleic acid, or the cell to a patient in need thereof, preferably wherein the cancer is Multiple Myeloma.

14. The CD229 targeting-moiety, the CAR, the nucleic acid, or the cell, for use according to claim 13, wherein said use is in combination with an anti BCMA therapy.

15. The CD229 targeting-moiety, the CAR, the nucleic acid, or the cell, for use according to claim 14, wherein the anti BCMA therapy comprises administering a CAR against BCMA.
